# EUROPÄISCHE PATENTSCHRIFT

(11) **EP 4 157 408 B1**
(45) Veröffentlichungstag und Bekanntmachung des Hinweises auf die Patenterteilung: **26.03.2025**
(21) Anmeldenummer: 21726885.3
(22) Anmeldetag: 17.05.2021
(51) Int. Cl.: A61M 16/00, A61B 5/08

(54) **BEATMUNGSVORRICHTUNG ZUR BESTIMMUNG ZUMINDEST DER GEWEBSRELATIERTEN RESISTANCE IM ATEMWEG**
VENTILATOR AND METHOD FOR DETERMINING AT LEAST THE TISSUE-RELATED RESISTANCE IN THE RESPIRATORY TRACT
APPAREIL RESPIRATOIRE ET PROCÉDÉ POUR DÉTERMINER AU MOINS LA RÉSISTANCE LIÉE AUX TISSUS DANS LES VOIES AÉRIENNES

(30) Priorität: 27.05.2020 DE 102020114209
(43) Veröffentlichungstag der Anmeldung: 05.04.2023
(73) Patentinhaber: Ventinova Technologies B.V., 5652 BJ Eindhoven (NL)
(72) Erfinder: ENK, Dietmar, 48653 Coesfeld (DE); BARNES, Thomas Heinrich, Warlingham Surrey CR6 9LB (GB)
(74) Vertreter: karo IP
(86) Internationale Anmeldenummer: PCT/EP2021/062937
(87) Internationale Veröffentlichungsnummer: WO 2021/239484

(56) Entgegenhaltungen:
- WO-A1-2017/148639
- US-A1- 2019 083 726

## Beschreibung

Die Erfindung betrifft eine Beatmungsvorrichtung zur (differenzierten) Messung bzw. Bestimmung von Kennwerten bzw. zumindest der gewebsrelatierten Resistance im Atemweg eines Patienten, ggf. zusätzlich zur Messung bzw. Bestimmung der atemwegsrelatierten Resistance und zur Ermittlung des (globalen) alveolären Druckes bzw. Druckverlaufes (in den Lungenbläschen) eines Patienten. WO2017/14639 beschreibt ein Beatmungsvorrichtung, wobei der Fluidstrom nicht gestoppt wird zur Bestimmung von Kennwerten.

Der inspiratorische Spitzendruck oder "peak inspiratory pressure" (PIP) bezeichnet den höchsten positiven Druck [in mbar, also Millibar], der während der Einatmung (Inspiration) künstlich im Atemweg erzeugt wird.

Der endinspiratorische (Plateau-)Druck ist der Druck, der am Ende der Inspiration im Atemweg gemessen wird.

Der endexspiratorische (Plateau-)Druck, der nach Abschluss der Ausatmung (Exspiration) im Atemweg gehalten wird, ist vorzugsweise positiv und wird daher auch als positiver endexspiratorischer Druck oder "positive end-expiratory pressure" (PEEP) bezeichnet. Im Folgenden wird immer auf den PEEP verwiesen.

Die Compliance [in ml / mbar, also Milliliter / Millibar] ist ein Maß für die Dehnbarkeit oder auch Druckelastizität des Lunge-Brustkorb-Systems eines Patienten. Unter Beatmungsbedingungen wird für die Berechnung der sogenannten statischen Compliance das Tidalvolumen (V_{T}) [in ml], das das während einer Inspiration zugeführte Luftvolumen bezeichnet, und die Differenz von endinspiratorischem (Plateau-)Druck (z. B. P_{I1}) und endexspiratorischem (Plateau-)Druck (z. B. P_{E1}) herangezogen.

Hingegen wird die sogenannte dynamische Compliance auf der Basis des V_{T} und der Differenz von PIP (z. B. P_{I2}) und PEEP (z. B. P_{E2}) [in mbar] berechnet. Die Druckdifferenz ist daher bei der dynamischen Compliance regelmäßig größer oder zumindest gleich groß wie die Druckdifferenz bei der statischen Compliance. Da die Compliance im Allgemeinen ein sich - mit sich änderndem Druck (P) und Volumen (V) - änderndes Verhältnis von Druck und Volumen aufweist, stellt sie sich in einem Druck-Volumen-Diagramm als Kurve dar.

Die Compliance gibt also an, wie viel Fluid (z. B. Atemgas, also ein Luftvolumen), also ein delta V, in den mindestens einen Atemweg eingeführt oder aus dem Atemweg entfernt wird, so dass sich ein Druck in dem Atemweg um eine Druckdifferenz delta P verändert. Während mindestens eines Beatmungsvorgangs (Inspiration, also dem Zuführen von Fluid in den Atemweg; Exspiration, also dem Abführen von Fluid aus dem Atemweg) kann ein Verlauf der Compliance-Kurve ermittelt oder zusätzlich abgeschätzt werden (z. B. aufgrund von Erfahrungswerten). Dabei kann insbesondere der Teilbereich der Compliance-Kurve ermittelt werden, bei dem ein bestimmtes Volumen (ggf. V_{T}) in einem möglichst kleinen Druckintervall zugeführt werden kann.

Ein Anwender oder vorzugsweise eine (automatisch arbeitende) Steuereinrichtung einer Beatmungsvorrichtung kann so, unter Berücksichtigung eines ermittelten oder zusätzlich abgeschätzten Verlaufs zumindest eines Teilbereichs einer Compliance-Kurve in einem Druck-Volumen-Diagramm, eine Lage eines Druckintervalls mit den Drücken P_{I} und P_{E} bestimmen und diese Drücke dann an der Beatmungsvorrichtung einstellen (z. B. PIP als P_{I} und PEEP als P_{E}), so dass zumindest ein Beatmungsvorgang, also eine Inspiration und / oder eine Exspiration, zwischen diesen Drücken P_{I} und P_{E} erfolgt und ein Betrag der Compliance dieses Beatmungsvorgangs möglichst groß ist.

Die fortlaufende Beatmung sollte dabei so eingestellt werden, dass ein für eine Normoventilation (also die adäquate Elimination bzw. Abatmung von Kohlendioxid) erforderliches Minutenvolumen (also V_{T} • Beatmungsfrequenz [/min, also Beatmungsvorgänge pro Minute]) möglichst klein ist und bei möglichst maximaler Compliance zu- und abgeführt werden kann.

Im Gegensatz zur statischen Compliance fließen in die dynamische Compliance zwangsläufig auch die während der In- bzw. Exspiration zu überwindenden Widerstände (im weitesten Sinne) einschließlich von Effekten der sogenannten Beatmungshistorie (also wie die Lunge beatmet wurde) mit ein. Letzteres ergibt sich aus dem Umstand, dass es sich bei der Lunge um ein viskoelastisches Organ handelt, dessen mechanische Eigenschaften davon abhängen, wie es bewegt wird bzw. wurde.

Die Resistance (gemessen in mbar / l / s; also Millibar / Liter / Sekunde bzw. mbar • s / l, also Millibar • Sekunde / Liter) beschreibt die während der In- bzw. Exspiration zu überwindenden Widerstände und gibt den Druck an, der für den Gasfluss (Fluidstrom) und damit die Volumenänderung (in der Lunge) pro Zeit notwendig ist.

Bei einem beatmeten Patienten wird die Resistance typischerweise während der Inspiration durch Messung der Druckdifferenz zwischen inspiratorischem Spitzendruck (PIP) und endinspiratorischem (Plateau-)Druck in Relation zum mittleren inspiratorischen Fluss (inspiratorischen Fluidstrom) abgeschätzt. Voraussetzung für die Messung ist ein Stopp des inspiratorischen Flusses.

So ergibt sich z. B. aus einer Druckdifferenz zwischen inspiratorischem Spitzendruck (P_{I2}) und endinspiratorischem (Plateau-)Druck (P_{E2}) von 2 mbar bei einem mittleren inspiratorischen Fluss von 18 l / min [Liter / Minute] eine (inspiratorische) Resistance von 6,66 mbar / l / s; also 2 mbar 18 l / min bzw. 2 mbar • 60 s l 18 l.

In konventionellen Beatmungsgeräten wird die Resistance zumeist mit diesem Verfahren bestimmt. Daneben gibt es verschiedene andere, auf intermittierenden oder superponierten Messungen beruhende Verfahren zur Bestimmung der Resistance.

Um die Eigenschaften einer beatmeten Lunge vollumfänglich erfassen und beschreiben zu können, ist neben einer präzisen Messung der dynamischen Compliance auch eine genaue Bestimmung der in- und exspiratorischen Resistance wünschenswert. Dies ist eine Voraussetzung, um insbesondere kritisch (lungen-) kranke Patienten individualisiert und möglichst schonend im Bereich optimaler Compliance beatmen zu können, also zwischen einem sogenannten unteren Inflektionspunkt, an dem die Compliance inspiratorisch im Sinne optimaler Rekrutierung des Lungengewebes maximal zunimmt und einem sogenannten oberen Inflektionspunkt, an dem die Compliance inspiratorisch aufgrund zunehmender Überdehnung im Lungengewebe maximal abnimmt.

Die Resistance ergibt sich aber nicht nur aus dem gasflussabhängigen Widerstand der Luftwege (atemwegsrelatiert; z. B. Querschnitt der Atemwege, Turbulenzen), sondern auch aus Widerständen im Gewebe (gewebsrelatiert; z. B. durch Massenträgheit, Scherung, Friktion, Viskoelastizität).

Massenträgheitseffekte spielen insbesondere zu Beginn der Inspiration und Exspiration eine Rolle, wenn durch die Zu- bzw. Abnahme des Lungenvolumens Gewebe (sowohl der Lunge selbst als auch umgebendes/ angrenzendes Gewebe) beschleunigt und abgebremst werden muss. Zu Scherung kann es innerhalb (insbesondere funktionell inhomogenen) Lungengewebes kommen (sogenannter "shear stress"), zu Friktion hingegen an Grenzflächen wie z. B. der Verschiebeschicht von Brust- und Lungenfell (Pleura parietalis und Pleura visceralis), die inspiratorisch zudem von der (Flächen-)Größe zu- und exspiratorisch von der (Flächen-)Größe wieder abnimmt. Viskoelastische Effekte ergeben sich u.a. aus einem in- und exspiratorisch unterschiedlichen Blutvolumen in der Lungenstrombahn, wodurch die Lunge unterschiedlich resistiv wird.

Innerhalb der Lunge gibt es nicht nur unterschiedlich kompliante (dehnbare) Lungenkompartimente, sondern auch bezüglich der Resistance finden sich Lungenkompartimente, die eine niedrigere oder höhere atemwegs- oder gewebsrelatierte Resistance aufweisen. Daraus ergibt sich notwendigerweise, dass man von außen immer nur ein globales Bild der Compliance und der Resistance bekommt.

Eine Differenzierung der Resistance in einen atemwegs- und gewebsrelatierten Teil ist aber klinisch interessant: Auf der Basis des atemwegsrelatierten Teils kann grundsätzlich der (globale) alveoläre Druckverlauf (in den Lungenbläschen) berechnet werden. Zudem ist eine erhöhte atemwegsrelatierte Resistance (im Gegensatz zum gewebsrelatierten Teil) einer medikamentösen Therapie zugänglich. Hingegen weist die gewebsrelatierte Resistance auf Veränderungen im (Lungen-)Gewebe hin. Damit bietet sich die gewebsrelatierte Resistance als diagnostischer, therapeutischer oder sogar prognostischer Parameter an.

In der Literatur wird der Anteil der gewebsrelatierten Resistance an der auf (inspiratorischem) Spitzendruck gemessenen (Gesamt-)Resistance auf etwa 25 % geschätzt, d. h. etwa 75 % der (Gesamt-)Resistance entfällt auf den atemwegsrelatierten Teil. Diesen Angaben liegen zumeist invasive Messverfahren (z. B. Ösophagusdruckmessungen) zugrunde.

Aufgabe der vorliegenden Erfindung ist es, die mit Bezug auf den Stand der Technik angeführten Probleme (zumindest teilweise) zu lösen. Insbesondere soll eine Beatmungsvorrichtung und ein Verfahren zumindest zur Messung bzw. Bestimmung der gewebsrelatierten Resistance im Atemweg eines Patienten, vorzugsweise zur (differenzierten) Messung bzw. Bestimmung der atemwegs- und gewebsrelatierten Resistance und zur Ermittlung des (globalen) alveolären Druckes bzw. Druckverlaufes, mit einer Beatmungsvorrichtung vorgeschlagen werden. Insbesondere soll eine Beatmungsvorrichtung vorgeschlagen werden, durch die Kennwerte festlegbar und bestimmbar sind.

Zur Lösung dieser Aufgaben trägt eine Beatmungsvorrichtung mit den Merkmalen gemäß Patentanspruch 1 und ein Verfahren mit den Merkmalen gemäß Patentanspruch 12 bei. Vorteilhafte Weiterbildungen sind Gegenstand der abhängigen Patentansprüche. Die in den Patentansprüchen einzeln aufgeführten Merkmale sind in technologisch sinnvoller Weise miteinander kombinierbar und können durch erläuternde Sachverhalte aus der Beschreibung und / oder Details aus den Figuren ergänzt werden, wobei weitere Ausführungsvarianten der Erfindung aufgezeigt werden.

Es wird eine Beatmungsvorrichtung vorgeschlagen, zumindest umfassend eine Gaszuführeinrichtung und eine Gasabführeinrichtung, zum Zuführen eines (inspiratorischen) Fluidstroms hin zu einem Atemweg eines Patienten und zum Abführen eines (exspiratorischen) Fluidstroms aus dem Atemweg (des Patienten) zurück in die Beatmungsvorrichtung oder an eine Umgebung, einen Drucksensor zur Erfassung eines Druckes in dem Atemweg sowie eine Steuereinrichtung zum Betreiben der Beatmungsvorrichtung. Ein Fluidstrom ist zumindest während eines Inspirationsvorgangs und eines Exspirationsvorgangs auf einen konstanten Wert einstellbar. Die Steuereinrichtung ist zum Betreiben der Beatmungsvorrichtung und zur Durchführung eines Verfahrens, insbesondere eines Messverfahrens, eingerichtet, das zumindest die folgenden Schritte umfasst:
a) Durchführen eines Inspirationsvorgangs mit einem konstanten ersten Fluidstrom mittels der Gaszuführeinrichtung,
b) Stoppen des (inspiratorischen) ersten Fluidstroms mittels der Gaszuführeinrichtung zu einem ersten Zeitpunkt, und dabei
c) (Messen oder) Bestimmen einer ersten Druckdifferenz zwischen einem zum ersten Zeitpunkt des Stoppens vorliegenden ersten Druck und einem sich nach einem Zeitintervall einstellenden zweiten Druck mittels des Drucksensors; sowie
d) Durchführen eines Exspirationsvorgangs mit einem konstanten zweiten Fluidstrom mittels der Gasabführeinrichtung,
e) Stoppen des (exspiratorischen) zweiten Fluidstroms mittels der Gasabführeinrichtung zu einem zweiten Zeitpunkt, und dabei
f) (Messen oder) Bestimmen einer zweiten Druckdifferenz zwischen einem zum zweiten Zeitpunkt des Stoppens vorliegenden dritten Druck und einem sich nach einem Zeitintervall einstellenden vierten Druck mittels des Drucksensors;
g) Festlegen und Bereitstellen (bzw. Bilden) einer Differenz aus der ersten Druckdifferenz und der zweiten Druckdifferenz als ein erster Kennwert, der zur Bestimmung zumindest einer gewebsrelatierten Resistance des Patienten verwendbar ist bzw. verwendet wird.

Vorzugsweise ist der erste Fluidstrom über den gesamten Inspirationsvorgang, insbesondere aber in der zweiten Hälfte des Inspirationsvorgangs, konstant. Vorzugsweise ist der zweite Fluidstrom über den gesamten Exspirationsvorgang, insbesondere aber in der zweiten Hälfte des Exspirationsvorgangs, konstant. Insbesondere sind der (inspiratorische) erste Fluidstrom und der (exspiratorische) zweite Fluidstrom betragsgleich.

Insbesondere bedeutet konstant oder betragsgleich hier, dass Abweichungen von bis zu 10 %, bevorzugt bis zu 5 %, besonders bevorzugt bis höchstens 1 %, gegenüber dem höchsten Wert des Fluidstroms vorliegen können.

Insbesondere erfolgt bei der hier vorgeschlagenen Beatmungsvorrichtung eine Beatmung des Patienten ausschließlich über die Beatmungsvorrichtung. Insbesondere wird also der Atemweg des Patienten ausschließlich über die Beatmungsvorrichtung mit dem Fluidstrom beaufschlagt (während der In- und Exspiration. Es liegt also insbesondere kein Fluidstrom vor, der nicht von der Beatmungsvorrichtung initiiert oder generiert wird. Insbesondere umfasst die Beatmungsvorrichtung dafür ein Lumen für die Inspiration und ein Lumen für die Exspiration. Insbesondere ist ein gemeinsames Lumen (z. B. ein Beatmungskatheter) vorgesehen, so dass der Fluidstrom nur durch ein Lumen dem Atemweg zugeführt oder aus dem Atemweg abgeführt wird.

Insbesondere ist der Drucksensor endotracheal (also in der Luftröhre) angeordnet. Damit kann der Druck innerhalb des Atemwegs des Patienten ermittelt werden.

Insbesondere ist der Drucksensor am distalen Ende eines Beatmungskatheters angeordnet, der sich als Bestandteil der Beatmungsvorrichtung in dem Atemweg des Patienten befindet.

Der Drucksensor kann ggf. auch nicht endotracheal, sondern beabstandet vom Patienten angeordnet sein. Der tracheale Druck sollte dann rechnerisch bestimmbar sein. Allerdings können sich bei einer derartigen Anordnung des Drucksensors Ungenauigkeiten ergeben, die die hier beschriebenen Messungen beeinträchtigen können.

Der Beatmungskatheter weist insbesondere ein Totraumvolumen (also das Volumen, dass während eines Inspirations- oder Exspirationsvorgangs in dem Beatmungskatheter verbleibt) von höchstens 100 ml auf, insbesondere von höchstens 50 ml.

Es ist beobachtet worden, dass sich die (Gesamt-)Resistance bei Zunahme des V_{T} aber gleichem Gaszufluss erhöht, mutmaßlich durch Zunahme der gewebsrelatierten Resistance. Dies erscheint aus folgendem Grund plausibel: Der geringe Druckfall von den kleinen Bronchien bzw. Bronchiolen (mit glatter Muskulatur), die maßgeblich die atemwegsrelatierte Resistance bestimmen, zu den Alveolen (Lungenbläschen) macht eine von den Beatmungsdrücken weitgehend unabhängige atemwegsrelatierte Resistance annehmbar, da höhere Drücke in den kleinen Bronchien bzw. Bronchiolen (mit glatter Muskulatur) notwendigerweise auch mit höheren Drücken in den abhängigen, die kleinen Bronchien bzw. Bronchiolen (mit glatter Muskulatur) umgebenden Alveolen einhergehen.

Insbesondere scheint die atemwegsrelatierte Resistance während des Inspirationsvorgangs aber eher leicht abzunehmen. Dies kann man durch eine (wenngleich geringe) Querschnittsvergrößerung der Bronchien bei steigendem Druck erklären, was den widerstandserhöhenden Effekt einer während der Inspiration zunehmenden Länge der kleinen Bronchien bzw. Bronchiolen (mit glatter Muskulatur) mehr als kompensiert. Diesbezüglich sei auf das Hagen-Poiseuille-Gesetz verwiesen, das beschreibt, dass sich Änderungen des Radius in der vierten Potenz, Änderungen der Länge aber lediglich proportional auf den Gasfluss (Fluidstrom) auswirken.

Auch wenn notwendigerweise die Höhe des Druckfalls von den kleinen Bronchien bzw. Bronchiolen (mit glatter Muskulatur) zu den Alveolen gasflussabhängig ist und es somit bei höheren Gasflüssen zu einer relativ stärkeren Dehnung und damit Querschnittsvergrößerung der kleinen Bronchien bzw. Bronchiolen (mit glatter Muskulatur) kommen kann, so führen höhere Atemwegsdrücke also allenfalls zu einer Abnahme, nicht aber Zunahme der atemwegsrelatierten Resistance.

Es ist daher plausibel anzunehmen, dass die Zunahme der inspiratorischen (Gesamt-)Resistance bei höheren Tidalvolumina aber gleichem Gaszufluss (Fluidstrom während Inspiration) maßgeblich auf einer Zunahme der gewebsrelatierten Resistance beruht. Umgekehrt darf man erwarten, dass bei exspiratorischem Abfluss des Atemgases (Fluidstrom während Exspiration) und dadurch abnehmendem Lungenvolumen der Anteil der gewebsrelatierten Resistance an der (Gesamt-)Resistance wieder abnimmt und bei vollständiger Exspiration schließlich (nahezu) verschwindet bzw. bei PEEP ein Minimum erreicht.

Insbesondere gilt also für Schritt g) die Annahme bzw. wird für das Verfahren durch die Steuereinrichtung festgelegt, dass die gewebsrelatierte Resistance im endexspiratorischen Zustand vernachlässigbar und im endinspiratorischen Zustand maximal ist.

Insbesondere ist mit der ausschließlichen Durchführung der Schritte d) bis f), und wenn der dritte Druck einem endexspiratorischen Druck entspricht, eine atemwegsrelatierte Resistance des Patienten bestimmbar (da die gewebsrelatierte Resistance im endexspiratorischen Zustand vernachlässigbar ist). Insbesondere ist es also möglich, bei endexspiratorischem Druck (als drittem Druck) nur die Schritte d) bis f) einmalig, oder vorzugsweise während der Beatmung wiederholt durchzuführen, um so die atemwegsrelatierte Resistance (direkt) zu ermitteln.

Insbesondere ist mit der Durchführung der Schritte d) bis f), und wenn der dritte Druck einem endexspiratorischen Druck entspricht, ein zweiter Kennwert festgelegt und bereitgestellt und damit eine atemwegsrelatierte Resistance des Patienten bestimmbar.

Insbesondere gilt für Schritt g) zusätzlich die Annahme bzw. wird für das Verfahren durch die Steuereinrichtung festgelegt, dass die gewebsrelatierte Resistance während des Inspirationsvorgangs zwischen dem endexspiratorischen Zustand und dem endinspiratorischen Zustand (annähernd) linear zunimmt und während des Exspirationsvorgangs zwischen dem endinspiratorischen Zustand und dem endexspiratorischen Zustand wieder (annähernd) linear abnimmt.

Insbesondere ist mit der Steuereinrichtung in Schritt g) auch eine atemwegsrelatierte Resistance (siehe nachstehendes 1. Rechnungsbeispiel) und (durch Umrechnung auf den bekannten konstanten Fluidstrom) der in- und exspiratorische Druckfall bestimmbar.

Somit wird hier insbesondere angenommen bzw. für das Verfahren durch die Steuereinrichtung festgelegt, dass die endexspiratorisch gemessene (Gesamt-)Resistance mehr oder weniger nur atemwegsrelatiert und damit niedriger ist als die nach Erreichen des Spitzendruckes (PIP) endinspiratorisch gemessene (Gesamt-)Resistance, in die auch die gewebsrelatierte Resistance eingeht. Diese entspricht wiederum der Differenz von in- und exspiratorisch gemessener (Gesamt-)Resistance.

Um die atemwegs- und / oder gewebsrelatierte Resistance bzw. den ersten Kennwert und / oder den zweiten Kennwert ermitteln zu können, ist insbesondere Folgendes nötig bzw. sinnvoll:
i. stabiler bzw. konstanter, insbesondere vom Betrag gleicher Gasfluss (Fluidstrom) während der Inspiration (Inspirationsvorgang) und Exspiration (Exspirationsvorgang). Es ist zu beachten, dass bei variierendem Fluidstrom (z. B. bei druck- oder volumenkontrollierter Beatmung) eine Umrechnung der gemessenen Resistance auf gleichen Gasfluss insbesondere bei (hoch)dynamischen Flussprofilen (mit nicht gleichem Gasfluss) zumindest komplex und aufwändig ist;
ii. idealerweise tracheale (in der Luftröhre) Messung des Druckes während der Inspiration; also z. B. des Druckabfalles nach Stopp des inspiratorischen Gaszuflusses (z. B. unmittelbar nach Erreichen des eingestellten inspiratorischen Spitzendruckes bzw. dem Erreichen des gewünschten zuzuführenden V_{T}) vom inspiratorischen Spitzendruck auf den endinspiratorischen (Plateau-)Druck;
iii. idealerweise tracheale (in der Luftröhre) Messung des Druckes während der Exspiration; also z. B. des Druckanstieges nach Stopp des exspiratorischen Gasabflusses (z. B. unmittelbar nach Rückkehr zum eingestellten endexspiratorischen Druck) vom endexspiratorischen Druck auf den endexspiratorischen (Plateau-)Druck.

Für das folgende 1. Rechnungsbeispiel wurde die erste Druckdifferenz im endinspiratorischen Zustand und die zweite Druckdifferenz im endexspiratorischen Zustand gemessen bzw. bestimmt. Im endinspiratorischen Zustand setzt sich die gemessene (Gesamt-)Resistance also aus einem Maximum der gewebsrelatierten Resistance und der (insbesondere über V_{T} konstanten) atemwegsrelatierten Resistance zusammen. Im endexspiratorischen Zustand umfasst die gemessene (Gesamt-)Resistance insbesondere nur die atemwegsrelatierte Resistance, da die gewebsrelatierte Resistance vernachlässigt werden kann.

### 1. Rechnungsbeispiel:

Eine als erste Druckdifferenz gemessene endinspiratorische (Gesamt-)Resistance (also Summe aus atemwegsrelatierter und gewebsrelatierter Resistance) von 8 mbar / l / s entspricht bei einem Gasfluss von 12 l / min (also 0,2 l / s) einer (Gesamt-)Resistance von 1,6 mbar 0,2 l / s. Wird als zweite Druckdifferenz endexspiratorisch eine (Gesamt-)Resistance von 4 mbar / l / s bei gleichem Fluss von 12 l / min gemessen (also 0,8 mbar 0,2 l / s), wobei endexspiratorisch angenommen wird, dass die Resistance dann nur atemwegsrelatiert und die gewebsrelatierte Resistance vernachlässigbar ist, so beträgt die atemwegsrelatierte Resistance also 0,8 mbar / 0,2 l / s (entsprechend der endexspiratorischen Messung) und die (maximale) gewebsrelatierte Resistance ebenfalls 0,8 mbar / 0,2 l / s, also 4 mbar / l / s (entsprechend der endinspiratorisch gemessene (Gesamt-)Resistance abzüglich der atemwegsrelatierten Resistance).

Dabei wird hier mittels des Drucksensors als erste Druckdifferenz zwischen dem endinspiratorischen ersten Druck und dem sich nach einem Zeitintervall einstellenden zweiten Druck, bei konstantem und bekanntem und bis zum Messen der ersten Druckdifferenz vorliegendem Fluidstrom von 12 l / min (also 0,2 l / s), ein Wert von 1,6 mbar gemessen.

Diese bestimmte bzw. gemessene erste Druckdifferenz kann aufgrund des bekannten Fluidstroms auf die endinspiratorische (Gesamt-)Resistance, also auf die atemwegsrelatierte und gewebsrelatierte Resistance umgerechnet werden, hier auf einen Wert von 8 mbar / l / s. Dabei kann der Wert von 8 mbar als auf einen Fluidstrom von 1 l / s normierter Wert der ersten Druckdifferenz verwendet werden.

Weiter wird mittels des Drucksensors als zweite Druckdifferenz (zweiter Kennwert) zwischen dem endexspiratorischen dritten Druck und dem sich nach einem Zeitintervall einstellenden vierten Druck, bei konstantem und gleichem und bis zum Messen der zweiten Druckdifferenz vorliegendem Fluidstrom von 12 l / min, ein Wert von 0,8 mbar gemessen.

Diese bestimmte bzw. gemessene zweite Druckdifferenz kann aufgrund des bekannten Fluidstroms auf die endexspiratorische (Gesamt-)Resistance, also auf die atemwegsrelatierte Resistance umgerechnet werden, hier also auf einen Wert von 4 mbar / l / s. Dabei kann der Wert von 4 mbar als auf einen Fluidstrom von 1 I / s normierter Wert der zweiten Druckdifferenz verwendet werden.

Die (maximale) gewebsrelatierte Resistance wird gemäß Schritt g) aus der Differenz zwischen der ersten Druckdifferenz und der zweiten Druckdifferenz bestimmt, hier: 1,6 mbar - 0,8 mbar = 0,8 mbar (bzw. normiert 8 mbar - 4 mbar = 4 mbar). Diese Differenz, also der Wert von 0,8 mbar, wird als erster Kennwert von der Steuereinrichtung festgelegt und bereitgestellt. Damit ergibt sich unter Berücksichtigung der in- und exspiratorisch konstanten und gleichen Fluidströme die (maximale) gewebsrelatierte Resistance zu 0,8 mbar / 0,2 l / s, also normiert 4 mbar / l / s (entsprechend der endinspiratorisch gemessenen (Gesamt-)Resistance abzüglich der atemwegsrelatierten Resistance).

Für Schritt g) und das Festlegen bzw. Bereitstellen der Differenz der Druckdifferenzen ist insbesondere eine Umrechnung der Druckdifferenzen auf normierte Druckdifferenzen erforderlich, d. h. die Druckdifferenzen sollten entweder (bevorzugt) bei gleichen Fluidströmen bestimmt bzw. gemessen oder darauf umgerechnet werden (siehe 1. Rechnungsbeispiel).

Unter der Annahme bzw. der Festlegung einer während der In- und Exspiration weitgehend gleichbleibenden atemwegsrelatierten Resistance und einer während der Inspiration sukzessiv (annähernd) linear zunehmenden und während der Exspiration sukzessiv (annähernd) linear wieder abnehmenden gewebsrelatierten Resistance (was bei einer Beatmung im Bereich optimaler bzw. maximaler Compliance und unter der Voraussetzung langsamer und gleichmäßiger Druck- und Volumenänderungen über die Zeit zulässig erscheint) kann man den (globalen) alveolären Druck bzw. Druckverlauf ableiten.

Hierfür muss man bzw. die Steuereinrichtung durch Umrechnung der atemwegsrelatierten Resistance (also der (Gesamt-)Resistance abzüglich der anteiligen gewebsrelatierten Resistance) auf den jeweiligen (aktuellen) Gasfluss zunächst den Druckfall ermitteln, der inspiratorisch für den Gasfluss von tracheal nach alveolär (d. h. von der Luftröhre in Richtung der Lungenbläschen) oder exspiratorisch von alveolär nach tracheal (also von den Lungenbläschen in Richtung der Luftröhre) verantwortlich ist. Insbesondere ergibt sich die Druckdifferenz (Druckfall) zwischen dem trachealen und dem alveolären Druck also aus der atemwegsrelatierten Resistance und dem konstanten Fluidstrom, also aus dem Produkt aus atemwegsrelatierter Resistance (als Multiplikator) und dem Fluidstrom (als Multiplikand) (im 1. Rechnungsbeispiel sind dies 0,8 mbar, nämlich 0,8 mbar / 0,2 I / s • 0,2 l / s bzw. 4 mbar / l / s • 12 l / min).

Dieser Druckfall wird dann in der Inspiration vom tracheal gemessenen Druck abgezogen (subtrahiert) bzw. in der Exspiration zum tracheal gemessenen Druck hinzugerechnet (addiert) und so der (globale) alveoläre Druck (zu einem gegebenen Zeitpunkt) bzw. Druckverlauf (über die Zeit) ermittelt.

Insbesondere ist mit der Steuereinrichtung zumindest zur Bestimmung der gewebsrelatierten Resistance eine Regressionsanalyse durchführbar, insbesondere eine lineare Regressionsanalyse. Dabei wird bevorzugt eine lineare Funktion zur Beschreibung der Veränderung der gewebsrelatierten Resistance zwischen dem endinspiratorischen Zustand und dem endexspiratorischen Zustand verwendet. Die Steuereinrichtung ist insbesondere zur Durchführung der Regressionsanalyse geeignet ausgeführt.

### 2. Rechnungsbeispiel:

Basierend auf (trachealer) Druckmessung folgt aus einer endinspiratorisch gemessenen ersten Druckdifferenz und einer so bestimmten (Gesamt-)Resistance von 6 mbar / I / s bei einem Gasfluss von 15 l / min (also 0,25 l / s) eine (Gesamt-)Resistance von 1,5 mbar / 0,25 l / s. Eine endexspiratorisch gemessene zweite Druckdifferenz und so bestimmte (Gesamt-)Resistance (entsprechend der atemwegsrelatierten Resistance) von 4 mbar / l / s entspricht bei gleichem Gasfluss 1 mbar / 0,25 l / s. Die maximale gewebsrelatierte Resistance entspricht also 0,5 mbar / 0,25 l / s. Bei halbem V_{T} (und bei linearer Regression hälftiger Anrechnung der gewebsrelatierten Resistance) errechnet sich in- wie auch exspiratorisch eine Druckdifferenz von jeweils 1,25 mbar, nämlich aus einer atemwegsrelatierten Resistance von 1 mbar / 0,25 l / s und einer gewebsrelatierten Resistance von 0,25 mbar / 0,25 l / s.

Wie im Zusammenhang mit dem 1. Rechnungsbeispiel erläutert, wird als erste Druckdifferenz ein Wert von 1,5 mbar mittels des Drucksensors gemessen. Als zweite Druckdifferenz wird ein Wert von 1 mbar gemessen. Aus der zweiten Druckdifferenz als zweitem Kennwert kann unmittelbar die normierte atemwegsrelatierte Resistance von 4 mbar / l / s abgeleitet werden. Damit kann aus der Differenz der Druckdifferenzen die maximale gewebsrelatierte Resistance von 0,5 mbar / 0,25 / s bzw. normiert 2 mbar / l / s bestimmt werden.

Insbesondere kann aus einer Messung bzw. Bestimmung von Druckdifferenzen während des Inspirationsvorgangs (und nicht erst bei Erreichen des inspiratorischen Spitzendruckes) und / oder des Exspirationsvorgangs (und nicht erst bei Erreichen des PEEP) zumindest die gewebsrelatierte Resistance über das jeweilige Druckintervall ermittelt werden. Dabei wird (z. B. mit der Steuereinrichtung) insbesondere die Lage der Zeitpunkte im Verhältnis zum endinspiratorischen Zustand und endexspiratorischen Zustand berücksichtigt und der Anteil der zum jeweiligen Zeitpunkt vorliegenden gewebsrelatierten Resistance z. B. anhand einer Regressionsanalyse bestimmt.

Insbesondere ist zumindest der zweite Druck oder der vierte Druck (durch die Steuereinrichtung) rechnerisch bestimmbar. Dabei kann angenommen werden, dass der Verlauf des bei inspiratorischer Messung auftretende Druckabfall und bei exspiratorischer Messung auftretende Druckanstieg jeweils typischerweise asymptotisch verläuft. Damit kann aus einem anfänglichen Verlauf des Druckabfalls bzw. Druckanstiegs der sich zu einem späteren Zeitpunkt einstellende Endwert des Druckes rechnerisch bestimmt oder zumindest abgeschätzt werden. Ggf. ist es auch möglich, diesen Druckabfall bzw. Druckanstieg für einen Patienten tatsächlich abzuwarten und so den Verlauf rechnerisch zu approximieren, so dass im weiteren Verlauf der Beatmung des Patienten der sich einstellende Endwert des Druckes anhand des approximativ ermittelten Verlaufs bestimmt werden kann.

Es sind verschiedene Beatmungsverfahren bekannt: Bei der (klassischen) volumenkontrollierten Beatmung ("Volume-Controlled Ventilation", VCV) sind das V_{T} und die Beatmungsfrequenz (und ggf. der PEEP) die primären Einstellparameter. Während der Insufflationsphase wird das V_{T} typischerweise mit einem konstanten Gasfluss (Fluidstrom) zugeführt. Der Gaszufluss ist in der Regel nicht direkt einstellbar und ergibt sich aus der Dauer der Insufflationsphase und der Dauer der nachfolgenden inspiratorischen Plateaudruckphase, in der kein Gas zugeführt wird. Bei der (klassischen) volumenkontrollierten Beatmung ergeben sich die Atemwegsdrücke abhängig vom eingestellten V_{T} sowie den pulmonalen Gegebenheiten des Patienten.

Bei der druckkontrollierten Beatmung ("Pressure-Controlled Ventilation", PCV) wird ein anfangs hoher inspiratorischer Gasfluss (Fluidstrom) kontinuierlich reduziert, während der Druckanstieg durch die Beatmungsvorrichtung erfasst wird. Zielparameter und Kontrollvariable ist also der inspiratorische Druck (und ggf. der PEEP). Das V_{T} ergibt sich aus den an der Beatmungsvorrichtung eingestellten Drücken und den pulmonalen Gegebenheiten des Patienten. Eine direkte Einstellung des inspiratorischen Gasflusses ist ebenfalls nicht möglich; dieser wird aber gemessen.

VCV und PCV setzen (wie auch alle hieraus abgeleiteten Beatmungsverfahren) auf eine unkontrollierte Exspiration. Damit hängt der exspiratorische Gasabfluss (Fluidstrom) von den Rückstellkräften des Brustkorb-Lunge-Systems und der exspiratorischen (Gesamt-)Resistance ab, ändert sich während der Exspiration ständig (nimmt sukzessiv ab) und läuft gegen Ende der Exspiration gegen Null.

Die Konstruktion einer (globalen) alveolären Druck-Zeit-Kurve erfordert daher nicht nur eine genaue, kontinuierliche exspiratorische Gasflussmessung, sondern auch komplexe Mathematik, mit der zu unterschiedlichen Zeiten während der Exspiration der jeweilige Druckfall von alveolär nach tracheal abgeschätzt werden kann (z. B. "Verfahren zur atemzugweisen kontinuierlichen Bestimmung der intratidalen dynamischen Atemmechanik mittels gleitender multipler Regressionsanalyse" gemäß DE 10 2006 025 809.6). Eine einfache Berechnung auf der Basis des mittleren exspiratorischen Gasabflusses ist hingegen sehr ungenau und somit untauglich.

Flusskontrollierte Ventilation ("Flow-Controlled Ventilation", FCV; z. B. DE 10 2016 103 678.1 und DE 10 2016 109 528.1) ist ein mittlerweile auch klinisch implementierter Beatmungsmodus, bei dem (im Gegensatz zu herkömmlichen Beatmungsvorrichtungen) der Gasfluss nicht nur inspiratorisch, sondern auch exspiratorisch kontrolliert und geregelt wird. Bei FCV entspricht der exspiratorische Gasabfluss insbesondere dem inspiratorischen Gaszufluss; dabei ergibt sich ein Verhältnis von In- und Exspiration von vorzugsweise 1:1. Der Gasfluss (Fluidstrom) ist stabil bzw. konstant (ändert sich also vom Betrag nicht relevant) und dabei vorzugsweise gerade so hoch, dass eine Normoventilation beim Patienten erreicht werden kann. Insbesondere zu Beginn der Exspiration, also ausgehend vom inspiratorischen Spitzendruck, wird der zweite Fluidstrom vorzugsweise reduziert (z. B. durch einen Widerstand). Während der Exspiration und insbesondere gegen Ende der Exspiration, also hin zum endexspiratorischen Druck, wird der zweite Fluidstrom dann zunehmend unterstützt (z. B. durch Saugwirkung).

Es ist nur ein weiteres, experimentelles (klinisch aber bislang nicht verfügbares) Beatmungsverfahren bekannt, bei dem der exspiratorische Gasabfluss moduliert werden kann: Bei "FLow-controlled EXpiration" (FLEX; s. Minerva Anestesiologica 80 (1): 19-28 (2014)) wird eine gewisse Kontrolle der Exspiration durch einen passiven, dynamischen Resistor erreicht, der im Ausatmungsschenkel eines herkömmlichen Beatmungsgerätes angeordnet ist und dessen Widerstand während der Exspiration sukzessiv erniedrigt wird. Abhängig von den Rückstellkräften des Brustkorb-Lunge-Systems und der exspiratorischen (Gesamt-)Resistance kann dieses System den Gasabfluss zwar modulieren, nicht aber einen (weitgehend) stabilen exspiratorischen Gasabfluss schaffen und halten. Auch ein I:E-Verhältnis von 1:1 und somit ein vom Betrag gleicher in- und exspiratorischer Gasfluss (Fluidstrom) ist nicht möglich.

Im Vergleich zu FLEX besteht der Vorteil von FCV darin, dass der exspiratorische Fluidstrom aktiv geregelt (im Sinne eines stabilen bzw. konstanten Gasflusses) und damit bekannt ist. Dies kann z. B. mit einem aktiven, dynamischen Resistor erreicht werden (z. B. Kombination eines Widerstandelementes mit einer Saugung, z. B. durch ein Gasstromumkehrelement, z. B. bekannt aus DE 10 2007 013 385.7). Dabei wird z. B. in einer (zeitlich) ersten Hälfte der Exspiration der durch die Rückstellkräfte des Brustkorb-Lunge-Systems besonders hohe Fluidstrom zunächst durch ein Widerstandselement verringert. In einer (zeitlich) zweiten Hälfte der Exspiration, wenn die Rückstellkräfte geringer werden und damit der Fluidstrom üblicherweise sukzessive abnehmen würde, wird der Fluidstrom von einer Saugung (z. B. einem Unterdruckanschluss oder ähnlichem) erhöht und insgesamt konstant gehalten.

Insbesondere in der (zeitlich) zweiten Hälfte der Exspiration ist der Gasabfluss (zweiter Fluidstrom) damit sehr stabil und kann vom Betrag insbesondere dem inspiratorischen Gaszufluss (ersten Fluidstrom) entsprechend eingestellt werden, also insbesondere über den gesamten Exspirationsvorgang hinweg.

Verglichen mit Beatmungsverfahren, die mit in- und / oder exspiratorisch dezelerierendem Gasfluss arbeiten (z. B. VCV, also volumenkontrollierte Beatmung mit nur inspiratorisch geregeltem Gasfluss, oder PCV, also druckkontrollierte Beatmung mit ebenfalls nur inspiratorisch geregeltem Gasfluss), schafft FCV optimale Bedingungen für die hier beschriebenen Messungen und Berechnungen.

Für eine möglichst einfache und genaue Berechnung des (globalen) alveolären Druckes bzw. Druckverlaufes ist ein jeweils stabiler bzw. konstanter, insbesondere vom Betrag gleicher, in- und exspiratorischer Fluidstrom bevorzugt, nicht zuletzt aufgrund von unvermeidlichen Latenzzeiten bei der sonst nötigen Gasflussmessung, Signalverarbeitung und nachfolgenden Regelung und Steuerung des Gasflusses. Diese Voraussetzungen erfüllt nur FCV.

Dabei ist insbesondere die zweite Hälfte der Inspiration und die zweite Hälfte der Exspiration besonders vorteilhaft, da hier bei FCV (sehr) stabile Fluidstrombedingungen vorliegen und die in der jeweils ersten Hälfte (insbesondere zu Beginn der Inspiration und Exspiration bzw. beim Wechsel von Inspiration zu Exspiration und von Exspiration zu Inspiration) relevanten Effekte (z. B. Massenträgheitseffekte) kaum mehr ins Gewicht fallen. Somit kann man aus der Umrechnung des (bevorzugt tracheal) gemessenen Druckes unter Berücksichtigung der ermittelten atemwegsrelatierten Resistance ein gutes Abbild des (globalen) alveolären Druckes bzw. Druckverlaufes erhalten.

Näherungsweise kann man den (globalen) alveolären Druck bzw. Druckverlauf auch auf der Basis der ermittelten (Gesamt-)Resistance abschätzen. Dabei sind aber wieder die speziellen Gasflussbedingungen von FCV (kontinuierlicher, konstanter, insbesondere vom Betrag gleicher Gasfluss während der In- und Exspiration) Voraussetzung für eine gute Näherung.

### 3. Rechnungsbeispiel:

Angesichts des typischerweise niedrigen Gasflusses bei FCV von maximal 15 l / min (also 0,25 l / s) ergibt sich selbst bei einer erhöhten (Gesamt-)Resistance von normiert 8 mbar / I / s (also 2 mbar / 0,25 l / s) durch Nichtberücksichtigung der gewebsrelatierten Resistance ein Fehler von maximal 1 mbar für den (globalen) alveolären Druck bzw. Druckverlauf (unter Annahme eines auf 50 % erhöhten Anteils der gewebsrelatierten Resistance an der (Gesamt-)Resistance).

Auch aus anderen (z. B. mechanischen und energetischen) Gründen ist eine Beatmung mit langsamen, gleichmäßigen Druck- und Volumenänderungen unter Nutzung des Bereiches individuell optimaler, also maximaler, Compliance sinnvoll.

FCV ist insbesondere für die kontrollierte, maximal lungenprotektive Beatmung, nicht aber zur Unterstützung von Spontanatmung gedacht, da hierfür deutlich höhere Gasflüsse notwendig sind.

Insbesondere durch einen möglichst niedrigen, stabilen und vom Betrag in- und exspiratorisch gleichen Fluidstrom können Unterschiede hinsichtlich der Gasverteilung in der Lunge (innerhalb des physikalisch Möglichen) minimiert werden. CT-Untersuchungen und elektrische Impedanz-Tomografie haben eine insgesamt bessere und homogenere Belüftung sowohl gesunder als auch kranker Lungen durch FCV bereits belegt.

Ziel der Erfindung ist es also, eine Beatmungsvorrichtung zu beschreiben, die eine einfache und (im Rahmen des physikalisch Möglichen) genaue Bestimmung von Kennwerten bzw. der atemwegs- und gewebsrelatierten Resistance und auf dieser Basis die Ermittlung des (globalen) alveolären Druckes bzw. Druckverlaufes während der kontrollierten Beatmung eines Patienten ermöglicht.

Bei der Beatmungsvorrichtung handelt es sich z. B. um ein Beatmungsgerät, das mit einem kontinuierlichen (ohne relevante Pausen), jeweils stabilen bzw. konstantem, vom Betrag in- und exspiratorisch gleichen Fluidstrom (und damit einem I:E-Verhältnis von typischerweise 1:1) vorzugsweise im Bereich optimaler bzw. maximaler Compliance beatmet. Dabei ist der Fluidstrom gerade so hoch, dass Normoventilation bzw. der gewünschte Grad der Kohlendioxidelimination bzw. -abatmung beim Patienten erreicht wird.

Ein Anwender oder vorzugsweise eine (automatisch arbeitende) Steuereinrichtung der Beatmungsvorrichtung kann, unter Berücksichtigung eines ermittelten oder zusätzlich abgeschätzten Verlaufs zumindest eines Teilbereichs einer Compliance-Kurve in einem Druck-Volumen-Diagramm, eine Lage eines Druckintervalls mit den Drücken P_{I} und P_{E} bestimmen und diese Drücke an der Beatmungsvorrichtung einstellen (z. B. PIP als P_{I} bzw. als ersten Druck und PEEP als P_{E} bzw. als dritten Druck), so dass zumindest ein Beatmungsvorgang, also eine Inspiration und / oder eine Exspiration, zwischen diesen Drücken P_{I} und P_{E} erfolgt und dadurch über das resultierende V_{T} ein Betrag der Compliance dieses Beatmungsvorgangs möglichst groß ist. Alternativ kann die Lage eines Druckintervalls mit den Drücken P_{I} und P_{E} auch in einem Volumen-Druck-Diagramm ermittelt werden. Der Beatmungsvorgang sollte zudem so eingestellt werden, dass ein für eine Normoventilation erforderliches Minutenvolumen bei möglichst maximaler Compliance zu- und abgeführt werden kann, da ein (optimal) großes V_{T} mit einer (optimal) niedrigen Beatmungsfrequenz die Effizienz der Kohlendioxidelimination erhöht und so der Atemweg bzw. das Gewebe des Patienten möglichst wenig belastet wird.

Auch wenn der in- und exspiratorische Fluidstrom vom Betrag unterschiedlich sein können, ist insbesondere nur eine Abweichung des aktuell vorliegenden Fluidstroms um höchstens 10 %, bevorzugt höchstens 5 %, besonders bevorzugt höchstens 1 %, jeweils während des Inspirationsvorgangs und während des Exspirationsvorgangs von einem eingestellten oder durchschnittlich vorliegenden Fluidstrom vorgesehen. Insbesondere ist eine entsprechende Abweichung auch zwischen Inspirationsvorgang und Exspirationsvorgang möglich. Insbesondere beträgt aber das Verhältnis zwischen Inspirationsvorgang und Exspirationsvorgang 1:1, ist also der Fluidstrom konstant und betragsgleich für den Inspirationsvorgang und den Exspirationsvorgang.

Die Beatmungsvorrichtung ist technisch insbesondere für eine kontinuierliche, bevorzugt tracheale Druckmessung ausgelegt und so programmiert bzw. programmierbar, dass wahlweise durch den Anwender oder in festen Zyklen während einer ausreichend langen Fluidstrompause sowohl während des Inspirationsvorgangs (erster Zeitpunkt), insbesondere nach Erreichen des inspiratorischen Spitzendruckes (PIP), der im sich dann anschließenden Zeitintervall auftretende Druckabfall als auch während des Exspirationsvorgangs (zweiter Zeitpunkt), insbesondere nach Erreichen des endexspiratorischen Druckes (PEEP), der im sich dann anschließenden Zeitintervall auftretende Druckanstieg gemessen bzw. bestimmt wird bzw. werden kann.

Dabei können die in- und exspiratorischen Messungen in denselben Beatmungszyklus (also ein Inspirationsvorgang und ein direkt folgender Exspirationsvorgang) fallen, auf zwei aufeinanderfolgende Beatmungszyklen verteilt oder vorzugsweise durch einige (normale) Beatmungszyklen getrennt sein.

Mit der Beatmungsvorrichtung bzw. mit dem Verfahren zur differenzierten Messung der atemwegs- und gewebsrelatierten Resistance und zur Ermittlung des (globalen) alveolären Druckes bzw. Druckverlaufes kann, ausgehend von diesen Druckmessungen und dem jeweiligen (bekannten) in- bzw. exspiratorischen Fluidstrom, die atemwegs- und gewebsrelatierte Resistance ermittelt und wahlweise ausgegeben werden (z. B. auf einem Display der Beatmungsvorrichtung).

Aus der atemwegsrelatierten Resistance und dem jeweiligen (bekannten) in- bzw. exspiratorischen Fluidstrom kann dann (auch während der Beatmung) der in- und exspiratorische Druckfall berechnet und wahlweise ausgegeben werden (z. B. auf dem Display der Beatmungsvorrichtung). Schließlich kann auch der (globale) alveoläre Druck bzw. Druckverlauf berechnet und wahlweise ausgegeben werden (z. B. auf dem Display der Beatmungsvorrichtung).

Mit der Beatmungsvorrichtung und / oder dem beschriebenen Verfahren kann die Bestimmung und Differenzierung der atemwegs- und gewebsrelatierten Resistance insbesondere mittels eines zwischenzeitlichen Stopps des Fluidstroms während der In- und Exspiration auch über Teilbereiche der (vorzugsweise optimalen, also maximalen) Compliance erfolgen.

Die Kombination einer Messung nach Erreichen des eingestellten inspiratorischen Spitzendruckes zum ersten Zeitpunkt mit einer zwischenzeitlichen Messung vor Erreichen des eingestellten endexspiratorischen Druckes zum zweiten Zeitpunkt erlaubt es insbesondere, die atemwegs- und gewebsrelatierte Resistance noch genauer zu bestimmen und zu differenzieren und dementsprechend auch den (globalen) alveolären Druck bzw. Druckverlauf noch genauer zu berechnen.

Gleiches leistet auch die Kombination einer zwischenzeitlichen Messung zum ersten Zeitpunkt vor Erreichen des eingestellten inspiratorischen Spitzendruckes mit einer Messung zum zweiten Zeitpunkt nach Erreichen des eingestellten endexspiratorischen Druckes.

Insbesondere bei zwischenzeitlichen Messungen, also während der Inspiration und vor Erreichen des inspiratorischen Spitzendruckes bzw. während der Exspiration und vor Erreichen des endexspiratorischen Druckes, sollte berücksichtigt werden, dass bei FCV insbesondere in der zweiten Hälfte der Inspiration und in der zweiten Hälfte der Exspiration (sehr) stabile bzw. konstante Gasflussbedingungen und daher dann besonders vorteilhafte Messbedingungen vorliegen.

Eine Messung mit einem intermittierend (also nur für den betreffenden Zyklus) niedrigeren bzw. höheren Spitzendruck und / oder einem intermittierend (also nur für den betreffenden Zyklus) niedrigeren bzw. höheren endexspiratorischen Druck ist ebenfalls möglich. Dies sollte aber vorzugsweise im Bereich maximaler Compliance geschehen, da es sonst bei zu hohem Spitzendruck zu einer (regionalen) Überdehnung bzw. bei zu niedrigem endexspiratorischen Druck zu einer (regionalen) Derekrutierung von Lungengewebe kommen kann.

Vorzugsweise wird der (globale) alveoläre Druck bzw. Druckverlauf auf der Basis der zeitlichen zweiten Hälfte der Inspiration und der zeitlichen zweiten Hälfte der Exspiration berechnet, da der Gaszufluss (inspiratorischer Fluidstrom von der Beatmungsvorrichtung hin zum Atemweg) bzw. Gasabfluss (exspiratorischer Fluidstrom aus dem Atemweg zurück in die Beatmungsvorrichtung oder in die Umgebung) dann jeweils (vom Betrag) sehr stabil ist und insbesondere in inhomogenen Lungen zu Beginn der In- und Exspiration bzw. beim Wechsel von Inspiration zu Exspiration und von Exspiration zu Inspiration auftretende Massenträgheitseffekte und hieraus resultierende Schereffekte kaum mehr relevant sind.

Insbesondere erfolgt zumindest in Schritt b) das Stoppen des (inspiratorischen) ersten Fluidstroms bei Erreichen eines inspiratorischen Spitzendruckes und / oder in Schritt e) das Stoppen des (exspiratorischen) zweiten Fluidstroms bei Erreichen eines endexspiratorischen Druckes (siehe z. B. auch das 1. Rechnungsbeispiel).

Insbesondere ergibt sich dabei bei der ersten Druckdifferenz (erster Druck entspricht inspiratorischem Spitzendruck) eine (Gesamt-)Resistance als Summe aus einer atemwegsrelatierten Resistance und einem Maximum einer gewebsrelatierten Resistance, wobei sich bei der zweiten Druckdifferenz (dritter Druck entspricht endexspiratorischem Druck) die (Gesamt-)Resistance (nur) aus der atemwegsrelatierten Resistance ergibt (weil die gewebsrelatierte Resistance vernachlässigbar ist bzw. nicht berücksichtigt wird).

Insbesondere ergibt sich die Druckdifferenz (Druckfall) zwischen dem trachealen und dem alveolären Druck aus der atemwegsrelatierten Resistance und dem konstanten Fluidstrom, also aus dem Produkt aus atemwegsrelatierter Resistance und dem Fluidstrom. Hieraus lässt sich insbesondere der alveoläre Druck bzw. (über die Zeit) der alveoläre Druckverlauf errechnen.

Alternativ kann auch auf der Basis der inspiratorischen (Gesamt-)Resistance (also ohne Berücksichtigung der gewebsrelatierten Resistance) der (globale) alveoläre Druck bzw. Druckverlauf schon recht gut abgeschätzt werden (siehe z. B. auch das 3. Rechnungsbeispiel).

Es wird weiter ein Verfahren zur Bestimmung zumindest einer gewebsrelatierten Resistance eines Patienten mit einer Beatmungsvorrichtung vorgeschlagen, insbesondere mit der beschriebenen Beatmungsvorrichtung.

Die Beatmungsvorrichtung umfasst zumindest eine Gaszuführeinrichtung und eine Gasabführeinrichtung, zum Zuführen eines (inspiratorischen) Fluidstroms hin zu einem Atemweg eines Patienten und zum Abführen eines (exspiratorischen) Fluidstroms aus dem Atemweg (des Patienten) zurück in die Beatmungsvorrichtung oder an eine Umgebung, einen Drucksensor zur Erfassung eines Druckes in dem Atemweg sowie eine Steuereinrichtung zum Betreiben der Beatmungsvorrichtung. Ein Fluidstrom ist zumindest während eines Inspirationsvorgangs und eines Exspirationsvorgangs auf einen konstanten Wert einstellbar. Die Steuereinrichtung ist zur Durchführung eines Verfahrens (bzw. eines Messverfahrens) geeignet ausgeführt bzw. eingerichtet, das zumindest die folgenden Schritte umfasst:
a) Durchführen eines Inspirationsvorgangs mit einem konstanten ersten Fluidstrom mittels der Gaszuführeinrichtung,
b) Stoppen des (inspiratorischen) ersten Fluidstroms mittels der Gaszuführeinrichtung zu einem ersten Zeitpunkt, und dabei
c) (Messen oder) Bestimmen einer ersten Druckdifferenz zwischen einem zum ersten Zeitpunkt des Stoppens vorliegenden ersten Druck und einem sich nach einem Zeitintervall einstellenden zweiten Druck mittels des Drucksensors; sowie
d) Durchführen eines Exspirationsvorgangs mit einem konstanten zweiten Fluidstrom mittels der Gasabführeinrichtung,
e) Stoppen des (exspiratorischen) zweiten Fluidstroms mittels der Gasabführeinrichtung zu einem zweiten Zeitpunkt, und dabei
f) (Messen oder) Bestimmen einer zweiten Druckdifferenz zwischen einem zum zweiten Zeitpunkt des Stoppens vorliegenden dritten Druck und einem sich nach einem Zeitintervall einstellenden vierten Druck mittels des Drucksensors;
g) Festlegen und Bereitstellen (bzw. Bilden) einer Differenz aus der ersten Druckdifferenz und der zweiten Druckdifferenz als ein erster Kennwert und Bestimmen zumindest einer gewebsrelatierten Resistance des Patienten.

Die obige (nicht abschließende) Einteilung der Verfahrensschritte in a) bis g) soll vorrangig nur zur Unterscheidung dienen und keine Reihenfolge und / oder Abhängigkeit erzwingen. Auch die Häufigkeit der Verfahrensschritte z. B. während der Einrichtung und / oder des Betriebes der Beatmungsvorrichtung kann variieren. Ebenso ist möglich, dass Verfahrensschritte einander zumindest teilweise zeitlich überlagern. Ganz besonders bevorzugt finden die Verfahrensschritte a) bis c) und d) bis f) jeweils abwechselnd nacheinander statt. Es ist aber auch möglich die Verfahrensschritte a) bis c) oder die Verfahrensschritte d) bis f) jeweils mehrmals zu wiederholen. Schritt g) kann insbesondere nach der einmaligen Durchführung der Schritte a) bis f) oder auch nach einer mehrmaligen Durchführung der Schritte a) bis f) oder zumindest der Schritte a) bis c) oder d) bis f) durchgeführt werden. Schritt g) kann bedingt sein und ggf. nur dann ausgeführt werden, wenn die Schritte a) bis f) zumindest einmal durchgeführt wurden.

Insbesondere werden die Schritte a) bis g) in der angeführten Reihenfolge durchgeführt.

Insbesondere werden zumindest die Schritte a) bis c) während eines Inspirationsvorgangs oder die Schritte d) bis f) während eines Exspirationsvorgangs jeweils zusammen mit dem Schritt g) wiederholt durchgeführt.

Insbesondere werden die Schritte a) bis c) während eines Inspirationsvorgangs und die Schritte d) bis f) während eines Exspirationsvorgangs jeweils zusammen mit dem Schritt g) wiederholt durchgeführt.

Es ist möglich, wie vorstehend beschrieben, dass die Schritte b) und e) im Rahmen des Verfahrens nur jeweils einmal zur Bestimmung der gewebsrelatierten Resistance durchgeführt werden. Dabei kann der erste Druck z. B. dem inspiratorischem Spitzendruck (PIP) und der dritte Druck dem endexspiratorischen Druck (PEEP) entsprechen, wobei aber auch andere Druckwerte für den ersten Druck und den dritten Druck verwendet werden können.

Insbesondere ist es möglich, bei endexspiratorischem Druck (als drittem Druck) nur die Schritte d) bis f) einmalig, oder vorzugsweise während der Beatmung wiederholt durchzuführen, um so einen zweiten Kennwert festzulegen bzw. bereitzustellen und damit die atemwegsrelatierte Resistance (direkt) zu ermitteln bzw. zu bestimmen.

Es ist auch möglich, den jeweiligen Fluidstrom mehrmals zu stoppen und bei unterschiedlichen Druckwerten die jeweilige Druckdifferenz zu bestimmen. Das Stoppen des jeweiligen Fluidstroms kann in einem Inspirationsvorgang bzw. Exspirationsvorgang desselben Beatmungszyklus oder in voneinander verschiedenen Beatmungszyklen durchgeführt werden.

Mit einer mehrmaligen Durchführung der genannten Schritte können die gesuchten Werte für die gewebsrelatierte Resistance und / oder atemwegsrelatierte Resistance, der Druckfall (von tracheal nach alveolär in der Inspiration bzw. von alveolär nach tracheal in der Exspiration) sowie der alveoläre Druck bzw. Druckverlauf genauer bestimmt werden.

Insbesondere gilt für Schritt g) die Annahme, dass die gewebsrelatierte Resistance im endexspiratorischen Zustand vernachlässigbar und im endinspiratorischen Zustand maximal ist und dazwischen während des Inspirationsvorgangs (annähernd) linear zunimmt und während des Exspirationsvorgangs (annähernd) linear abnimmt.

Es wird weiter eine Steuereinrichtung für eine Beatmungsvorrichtung, insbesondere für die beschriebene Beatmungsvorrichtung, vorgeschlagen, die zur Durchführung des beschriebenen Verfahrens (geeignet) ausgestattet, konfiguriert oder programmiert ist.

Die Ausführungen zur Beatmungsvorrichtung sind insbesondere auf das Verfahren und die Steuereinrichtung übertragbar und jeweils umgekehrt.

Weiter kann das beschriebene Verfahren auch von einem Anwender (teilweise) manuell oder einem (separaten) Computer bzw. mit einem Prozessor einer Steuereinrichtung halbautomatisch oder (voll)automatisch ausgeführt werden.

Es wird demnach auch ein System zur Datenverarbeitung vorgeschlagen, das einen Prozessor umfasst, der so angepasst, programmiert und konfiguriert ist, dass er das beschriebene Verfahren bzw. einen Teil der Schritte des Verfahrens (ggf. im Dialog mit einem Anwender) durchführt.

Es kann ein computerlesbares Speichermedium vorgesehen sein, das Befehle / Algorithmen umfasst, die bei der Ausführung durch einen Computer / Prozessor diesen veranlassen, das beschriebene Verfahren bzw. mindestens einen Teil der Schritte des Verfahrens (ggf. im Dialog mit einem Anwender) auszuführen.

Die Verwendung unbestimmter Artikel ("ein", "eine", "einer" und "eines"), insbesondere in den Patentansprüchen und in der diese wiedergebenden Beschreibung, ist als solche und nicht als Zahlwort zu verstehen. Entsprechend damit eingeführte Begriffe bzw. Komponenten sind somit so zu verstehen, dass diese mindestens einmal vorhanden sind und insbesondere aber auch mehrfach vorhanden sein können.

Vorsorglich sei angemerkt, dass die hier verwendeten Zahlwörter ("erste", "zweite", ...) vorrangig (nur) zur Unterscheidung von mehreren gleichartigen Gegenständen, Größen oder Prozessen dienen, also insbesondere keine Abhängigkeit und / oder Reihenfolge dieser Gegenstände, Größen oder Prozesse zueinander zwingend vorgeben. Sollte eine Abhängigkeit und / oder Reihenfolge erforderlich sein, ist dies hier explizit angegeben oder es ergibt sich offensichtlich für den Fachmann beim Studium der konkret beschriebenen Ausgestaltung. Soweit ein Bauteil mehrfach vorkommen kann ("mindestens ein"), kann die Beschreibung zu einem dieser Bauteile für alle oder ein Teil der Mehrzahl dieser Bauteile gleichermaßen gelten; dies ist aber nicht zwingend.

Die Erfindung sowie das technische Umfeld werden nachfolgend anhand der beiliegenden Figuren näher erläutert. Es ist darauf hinzuweisen, dass die Erfindung durch die angeführten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und Erkenntnissen aus der vorliegenden Beschreibung zu kombinieren. Insbesondere ist darauf hinzuweisen, dass die Figuren und insbesondere die dargestellten Größenverhältnisse nur schematisch sind. Es zeigen:
- Fig. 1:: eine Beatmungsvorrichtung im Betrieb;
- Fig. 2:: eine erste Variante des Verfahrens;
- Fig. 3:: eine zweite Variante des Verfahrens; und
- Fig. 4:: ein Druck-Volumen-Diagramm.

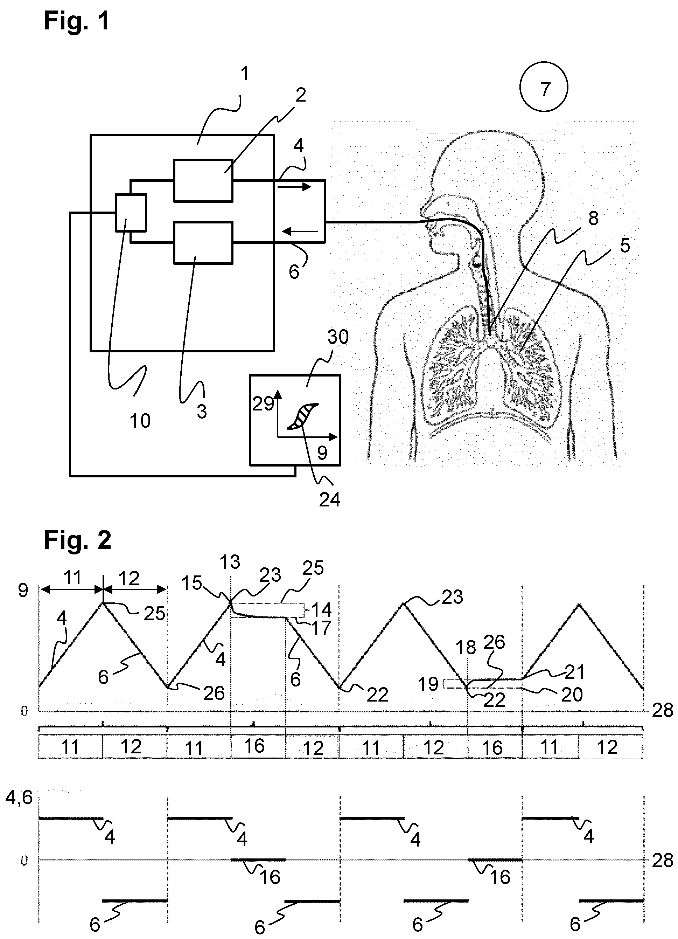

Die Fig. 1 zeigt eine Beatmungsvorrichtung 1 im Betrieb. Die Beatmungsvorrichtung 1 umfasst eine Gaszuführeinrichtung 2 und eine Gasabführeinrichtung 3, zum Zuführen eines (inspiratorischen) ersten Fluidstroms 4 hin zu einem Atemweg 5 eines Patienten und zum Abführen eines (exspiratorischen) zweiten Fluidstroms 6 aus dem Atemweg 5 zurück in die Beatmungsvorrichtung 1 oder in die Umgebung 7, einen Drucksensor 8 zur Erfassung eines Druckes 9 in dem Atemweg 5 sowie eine Steuereinrichtung 10 zum Betreiben der Beatmungsvorrichtung 1. Der Fluidstrom 4, 6 ist während eines Inspirationsvorgangs 11 und eines Exspirationsvorgangs 12 auf einen konstanten Wert einstellbar. Die Steuereinrichtung 10 ist zum Betreiben der Beatmungsvorrichtung 1 und zur Durchführung des Messverfahrens geeignet ausgeführt.

Der Drucksensor 8 ist endotracheal angeordnet. Der Drucksensor 8 befindet sich am distalen Ende eines Beatmungskatheters, der als Bestandteil der Beatmungsvorrichtung 1 in dem Atemweg 5 des Patienten angeordnet ist.

Die Beatmungsvorrichtung 1 umfasst weiter eine Visualisierungseinrichtung 30 (z. B. ein Display), auf der die (Gesamt-)Resistance, atemwegsrelatierte Resistance und gewebsrelatierte Resistance, insbesondere aber auch der aktuelle alveoläre Druck 9 über die Zeit 28 und / oder der Verlauf 24 des alveolären Druckes 9 und Volumens 29 (als Druck-Volumen-Kurve) darstellbar sind.

Fig. 2 zeigt eine erste Variante des Verfahrens. In der Fig. 2 sind mehrere Beatmungszyklen dargestellt. In dem oberen Teil der Fig. 2 ist ein Diagramm dargestellt, bei dem an der vertikalen Achse der Druck 9 aufgetragen ist. Auf der horizontalen Achse ist die Zeit 28 aufgetragen. In dem unteren Teil der Fig. 2 ist ein Diagramm dargestellt, bei dem an der vertikalen Achse der Fluidstrom 4, 6 aufgetragen ist. Auf der horizontalen Achse ist die Zeit 28 aufgetragen.

Im ersten Beatmungszyklus (ganz links) ist ein normaler FCV-Zyklus mit einem stabilen, in- und exspiratorisch (vom Betrag) gleichen Fluidstrom 4, 6 und somit einem Verhältnis von Inspirationsvorgang 11 und Exspirationsvorgang 12 von 1:1 dargestellt. Der zweite Beatmungszyklus (zweiter von links) und der dritte Beatmungszyklus (zweiter von rechts) sind Messzyklen: Bei dem zweiten Beatmungszyklus stoppt der erste Fluidstrom 4 bei Erreichen des eingestellten inspiratorischen Spitzendruckes 25 (erster Druck 15). Nachfolgend fällt der Druck 9 in dem Zeitintervall 16 auf einen endinspiratorischen (Plateau-)Druck, den zweiten Druck 17, ab. Die so ermittelbare erste Druckdifferenz 14 in Relation zum inspiratorischen ersten Fluidstrom 4 ergibt die (Gesamt-)Resistance (also die Summe aus atemwegsrelatierter und gewebsrelatierter Resistance). Bei dem dritten Beatmungszyklus stoppt der exspiratorische zweite Fluidstrom 6 bei Erreichen des eingestellten endexspiratorischen Druckes 26 (dritter Druck 20). Nachfolgend steigt der Druck 9 auf einen leicht höheren endexspiratorischen (Plateau-)Druck, den vierten Druck 21, an. Die so ermittelbare zweite Druckdifferenz 19 in Relation zum exspiratorischen zweiten Fluidstrom 6 ergibt die atemwegsrelatierte Resistance. Die gewebsrelatierte Resistance ist die Differenz aus den Druckdifferenzen 14, 19 in Relation zum in- bzw. exspiratorischen Fluidstrom 4, 6. Der vierte Beatmungszyklus (ganz rechts) ist wieder ein normaler FCV-Zyklus mit einem aufgrund der vorausgegangenen Messung und dem somit leicht erhöhten Startdruck leicht verkürzten Inspirationsvorgang 11.

Gemäß Schritt a) des Verfahrens bzw. des Messverfahrens erfolgt im zweiten Beatmungszyklus ein Durchführen eines Inspirationsvorgangs 11 mit einem konstanten ersten Fluidstrom 4. Gemäß Schritt b) erfolgt ein Stoppen des ersten Fluidstroms 4 zu einem ersten Zeitpunkt 13, und dabei gemäß Schritt c) ein Messen oder Bestimmen einer ersten Druckdifferenz 14 zwischen einem zum ersten Zeitpunkt 13 des Stoppens vorliegenden ersten Druck 15 und einem sich nach einem Zeitintervall 16 einstellenden zweiten Druck 17. Nachfolgend erfolgt ein Exspirationsvorgang 12. Im darauffolgenden dritten Beatmungszyklus erfolgt zunächst ein Inspirationsvorgang 11, wobei danach gemäß Schritt d) ein Durchführen eines Exspirationsvorgangs 12 mit einem konstanten zweiten Fluidstrom 6 erfolgt. Gemäß Schritt e) erfolgt ein Stoppen des zweiten Fluidstroms 6 zu einem zweiten Zeitpunkt 18, und dabei gemäß Schritt f) ein Messen oder Bestimmen einer zweiten Druckdifferenz 19 zwischen einem zum zweiten Zeitpunkt 18 des Stoppens vorliegenden dritten Druck 20 und einem sich nach einem Zeitintervall 16 einstellenden vierten Druck 21. Gemäß Schritt g) erfolgt ein Bilden der Differenz aus der ersten Druckdifferenz 14 und der zweiten Druckdifferenz 19 und Bestimmen zumindest einer gewebsrelatierten Resistance des Patienten.

Fig. 2 entspricht dem 1. Rechnungsbeispiel, bei dem die erste Druckdifferenz 14 im endinspiratorischen Zustand 23 und die zweite Druckdifferenz 19 im endexspiratorischen Zustand 22 gemessen wird. Im endinspiratorischen Zustand 23 setzt sich die gemessene (Gesamt-)Resistance also aus einem Maximum der gewebsrelatierten Resistance und der (insbesondere konstanten) atemwegsrelatierten Resistance zusammen. Im endexspiratorischen Zustand 22 umfasst die gemessene (Gesamt-)Resistance insbesondere nur die atemwegsrelatierte Resistance, da die gewebsrelatierte Resistance vernachlässigt wird.

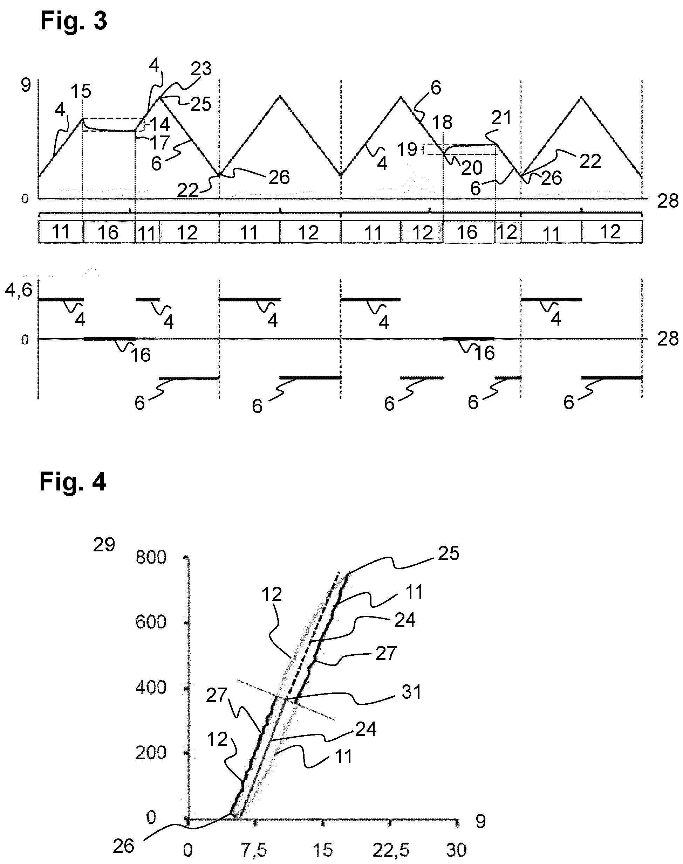

Fig. 3 zeigt eine zweite Variante des Verfahrens. Auf die Ausführungen zu Fig. 2 wird Bezug genommen.

In der Fig. 3 sind mehrere Beatmungszyklen dargestellt. In dem oberen Teil der Fig. 3 ist ein Diagramm dargestellt, bei dem an der vertikalen Achse der Druck 9 aufgetragen ist. Auf der horizontalen Achse ist die Zeit 28 aufgetragen. In dem unteren Teil der Fig. 3 ist ein Diagramm dargestellt, bei dem an der vertikalen Achse der Fluidstrom 4, 6 aufgetragen ist. Auf der horizontalen Achse ist die Zeit 28 aufgetragen.

Der zweite (zweiter von links) und der vierte Beatmungszyklus (ganz rechts) sind normale FCV-Zyklen mit einem stabilen, in- und exspiratorisch (vom Betrag) gleichen Fluidstrom 4, 6 und somit einem Verhältnis von Inspirationsvorgang 11 und Exspirationsvorgang 12 von 1:1. Der erste (ganz links) und vierte Beatmungszyklus sind Messzyklen: Bei dem ersten Beatmungszyklus stoppt der (inspiratorische) erste Fluidstrom 4 zwischenzeitlich vor Erreichen des eingestellten inspiratorischen Spitzendruckes 25 bei einem ersten Druck 15. Nachfolgend fällt der Druck 9 auf einen (zwischen-)inspiratorischen (Plateau-)Druck, den zweiten Druck 17, ab. Die erste Druckdifferenz 14 in Relation zum inspiratorischen ersten Fluidstrom 4 ergibt die (Gesamt-)Resistance (also die Summe aus atemwegsrelatierter und gewebsrelatierter Resistance) zu diesem ersten Zeitpunkt 13. Bei dem dritten Beatmungszyklus (zweiter von rechts) stoppt der (exspiratorische) zweite Fluidstrom 6 zwischenzeitlich vor Erreichen des eingestellten endexspiratorischen Druckes 26 bei einem dritten Druck 20. Nachfolgend steigt der Druck 9 auf einen leicht höheren (zwischen)exspiratorischen (Plateau)Druck, den vierten Druck 21, an. Die zweite Druckdifferenz 19 in Relation zum exspiratorischen zweiten Fluidstrom 6 ergibt die atemwegsrelatierte (Gesamt-)Resistance (also die Summe aus atemwegsrelatierter und gewebsrelatierter Resistance) zu diesem zweiten Zeitpunkt 18. Die gewebsrelatierte Resistance über die Druckdifferenz 14, 19 bei zwischenzeitlichem in- bzw. exspiratorischen Stopp des Fluidstroms 4, 6 ist die Differenz aus der ersten Druckdifferenz 14 und der zweiten Druckdifferenz 19 in Relation zum in- bzw. exspiratorischen Fluidstrom 4, 6.

Unter der Annahme einer während des Inspirationsvorgangs 11 und Exspirationsvorgangs 12 weitgehend gleichbleibenden atemwegsrelatierten Resistance und einer während des Inspirationsvorgangs 11 sukzessiv (annähernd) linear zunehmenden und während des Exspirationsvorgangs 12 sukzessiv (annähernd) linear wieder abnehmenden gewebsrelatierten Resistance (was bei einer Beatmung im Bereich optimaler bzw. maximaler Compliance und unter der Voraussetzung langsamer und gleichmäßiger Änderungen von Druck 9 und Volumen 29 über die Zeit 28 zulässig erscheint) kann man den (globalen) alveolären Druck 9 bzw. Verlauf 24 des alveolären Druckes 9 ableiten.

Hierfür muss man durch Umrechnung der atemwegsrelatierten Resistance auf den jeweiligen (aktuellen) Fluidstrom 4, 6 den Druckfall ermitteln, der inspiratorisch für den Gasfluss von tracheal nach alveolär (d. h. von der Luftröhre in Richtung der Lungenbläschen) bzw. exspiratorisch von alveolär nach tracheal (also von den Lungenbläschen in Richtung der Luftröhre) verantwortlich ist.

Mit der Steuereinrichtung 10 ist zumindest zur Bestimmung der gewebsrelatierten Resistance eine Regressionsanalyse durchführbar, insbesondere eine lineare Regressionsanalyse. Dabei wird eine lineare Funktion zur Beschreibung der Veränderung der gewebsrelatierten Resistance zwischen dem endinspiratorischen Zustand 23 und dem endexspiratorischen Zustand 22 verwendet. Die Steuereinrichtung 10 ist insbesondere zur Durchführung der Regressionsanalyse geeignet ausgeführt.

Damit kann aus einer Messung der Druckdifferenzen 14, 19 während des Inspirationsvorgangs 11 (und nicht erst bei Erreichen des inspiratorischen Spitzendruckes 25) und / oder des Exspirationsvorgangs 12 (und nicht erst bei Erreichen des endexspiratorischen Druckes 26) zumindest die gewebsrelatierte Resistance ermittelt werden. Dabei wird insbesondere die Lage der Zeitpunkte 13, 18 im Verhältnis zur Lage des endinspiratorischen Zustands 23 und des endexspiratorischen Zustands 22 berücksichtigt und der Anteil der zu diesem Zeitpunkt 13, 18 vorliegenden gewebsrelatierten Resistance z. B. anhand einer Regressionsanalyse bestimmt. In diesem Zusammenhang wird auf das vorstehend erwähnte 2. Rechnungsbeispiel verwiesen.

Fig. 4 zeigt ein Druck-Volumen-Diagramm. Auf der vertikalen Achse ist das Volumen 29 (in ml) aufgetragen. Auf der horizontalen Achse ist der Druck 9 (in mbar) aufgetragen. Auf die Ausführungen zu den Fig. 1 bis 3 wird verwiesen.

Der Fig. 4 liegt eine Druck-Volumen-Kurve eines optimal beatmeten Patienten zugrunde. Schon auf der Basis der inspiratorischen (Gesamt-)Resistance kann der (globale) alveoläre Druck 9 bzw. der Verlauf 24 des alveolären Druckes 9 recht gut abgeschätzt werden. Hierfür wird die jeweils zweite Hälfte 27 der in- und exspiratorischen Druck-Volumen-Kurve herangezogen, da hier bei FCV (sehr) stabile Fluidstrombedingungen vorliegen und die in der jeweils ersten Hälfte (insbesondere zu Beginn des Inspirationsvorgangs 11 und Exspirationsvorgangs 12 bzw. beim Wechsel von Inspirationsvorgang 11 zum Exspirationsvorgang 12 und wieder Inspirationsvorgang 11) relevanten Effekte (z. B. Massenträgheitseffekte und hieraus resultierende Schereffekte) kaum mehr ins Gewicht fallen.

In diesem Beispiel beträgt der in- und exspiratorische Fluidstrom 4, 6 jeweils 11 l / min (= 0,183 l / s). Die inspiratorische (Gesamt-)Resistance ist 5,7 mbar / I / s bzw. 1,05 mbar 0, 183 l / s (bezogen auf den Fluidstrom 4, 6). Die Breite der Druck-Volumen-Kurve in der Mitte (entlang der die Kurve schneidenden gestrichelten Linie) beträgt 2,1 mbar. Der mit 1,05 mbar abgeschätzte (maximale) inspiratorische Druckfall von tracheal nach alveolär bzw. dessen Verlauf 24 ist als gestrichelte Parallele zur geraden zweiten Hälfte 27 der inspiratorischen Druck-Volumen-Kurve (hier schwarz nachgezogen), also des Inspirationsvorgangs 11, eingezeichnet. Der gleich hoch angesetzte exspiratorische Druckfall von alveolär nach tracheal bzw. dessen Verlauf 24 ist entsprechend als durchgezogene Parallele zur geraden zweiten Hälfte 27 der exspiratorischen Druck-Volumen-Kurve (hier ebenfalls schwarz nachgezogen), also des Exspirationsvorgangs 12 eingezeichnet.

Zusammen geben die gestrichelte und die durchgezogene Parallele den (globalen) alveolären Druck 9 bzw. den Verlauf 24 des alveolären Druckes 9 während dieses Beatmungszyklus recht gut wieder.

Es fällt auf, dass die beiden Parallelen zusammen keine Gerade ergeben, sondern leicht gegeneinander verkippt sind. Dies bildet (stark vereinfacht) den mittleren Bereich einer S-förmig geschwungenen dynamischen Compliance-Kurve ab. Dabei zeigt der durch die beiden Parallelen gebildete Knickpunkt 31 die Inflektion (Wechsel der Krümmungsrichtung) korrekt an. Bei dem hier beschriebenen Verfahren zur Abschätzung des (globalen) alveolären Druckes 9 bzw. des Verlaufs 24 des alveolären Druckes 9 wird dieser insbesondere in der frühen Inspiration zwangsläufig etwas zu hoch ermittelt, da hierfür die höhere endinspiratorisch gemessene (Gesamt-)Resistance und nicht die niedrigere endexspiratorische (hauptsächlich atemwegsrelatierte) Resistance herangezogen wird. Dies ist aber nur dann relevant, wenn sich während des Inspirationsvorgangs 11 eine messtechnisch erfassbare gewebsrelatierte Resistance aufbaut, die dann während des Exspirationsvorgangs 12 abnimmt und zum Ende des Exspirationsvorgangs 12 wieder (nahezu) verschwindet.

Insbesondere wird also der (globale) alveoläre Druck 9 bzw. der Verlauf 24 des alveolären Druckes 9 auf der Basis der zweiten Hälfte 27 des Inspirationsvorgangs 11 und der zweiten Hälfte 27 des Exspirationsvorgangs 12 berechnet, da der erste Fluidstrom 4 von der Beatmungsvorrichtung 1 hin zum Atemweg 5 bzw. der zweite Fluidstrom 6 aus dem Atemweg 5 zurück in die Beatmungsvorrichtung 1 oder in die Umgebung 7 dann jeweils (vom Betrag) sehr stabil ist und insbesondere in inhomogenen Lungen zu Beginn des Inspirationsvorgangs 11 und Exspirationsvorgangs 12 bzw. beim Wechsel vom Inspirationsvorgang 11 zum Exspirationsvorgang 12 und vom Exspirationsvorgang 12 zum Inspirationsvorgang 11 auftretende Massenträgheitseffekte und hieraus resultierende Schereffekte kaum mehr relevant sind.

Mit der Beatmungsvorrichtung 1 bzw. mit dem Verfahren zur differenzierten Messung der atemwegs- und gewebsrelatierten Resistance und zur Ermittlung des (globalen) alveolären Druckes 9 bzw. des Verlaufs 24 des alveolären Druckes 9 kann, ausgehend von den beschriebenen Druckmessungen gemäß der Schritte c) und f) und dem jeweiligen (bekannten) in- bzw. exspiratorischen Fluidstrom 4, 6, die atemwegs- und gewebsrelatierte Resistance ermittelt und wahlweise ausgegeben werden.

Aus der atemwegsrelatierten Resistance und dem jeweiligen (bekannten) in- bzw. exspiratorischen Fluidstrom 4, 6 kann dann (auch während der Beatmung) der in- und exspiratorische Druckfall berechnet und wahlweise ausgegeben werden (z. B. auf dem Display der Beatmungsvorrichtung 1). Schließlich kann der (globale) alveoläre Druck 9 bzw. der Verlauf 24 des alveolären Druckes 9 berechnet und wahlweise ausgegeben werden (z. B. auf dem Display der Beatmungsvorrichtung 1).

Mit der Beatmungsvorrichtung 1 und / oder dem beschriebenen Verfahren kann die Bestimmung und Differenzierung der atemwegs- und gewebsrelatierten Resistance insbesondere mittels eines zwischenzeitlichen Stopps des Fluidstroms 4, 6 während des Inspirationsvorgangs 11 bzw. Exspirationsvorgangs 12 auch über Teilbereiche der (optimalen, also maximalen) Compliance erfolgen (siehe z. B. Fig. 3, erster Beatmungszyklus (ganz links) und Fig. 3, dritter Beatmungszyklus (zweiter von rechts)).

Die Kombination einer Messung nach Erreichen des eingestellten inspiratorischen Spitzendruckes 25 zum ersten Zeitpunkt 13 (siehe z. B. Fig. 2, zweiter Beatmungszyklus (zweiter von links) mit der im sich anschließenden Zeitintervall 16 auftretenden ersten Druckdifferenz 14) mit einer zwischenzeitlichen Messung vor Erreichen des eingestellten endexspiratorischen Druckes 26 zum zweiten Zeitpunkt 18 (siehe z. B. Fig. 3, dritter Beatmungszyklus (zweiter von rechts), mit der im sich anschließenden Zeitintervall 16 auftretenden zweiten Druckdifferenz 19) erlaubt es insbesondere, die atemwegs- und gewebsrelatierten Resistance noch genauer zu bestimmen und zu differenzieren und dementsprechend auch den (globalen) alveolären Druck 9 bzw. den Verlauf 24 des alveolären Druckes 9 noch genauer zu berechnen.

Gleiches leistet auch die Kombination einer zwischenzeitlichen Messung zum ersten Zeitpunkt 13 vor Erreichen des eingestellten inspiratorischen Spitzendruckes 25 (siehe Fig. 3, erster Beatmungszyklus (ganz links), mit der im sich anschließenden Zeitintervall 16 auftretenden ersten Druckdifferenz 14) mit einer Messung zum zweiten Zeitpunkt 18 nach Erreichen des eingestellten endexspiratorischen Druckes 26 (siehe z. B. Fig. 2, dritter Beatmungszyklus (zweiter von rechts), mit der im sich anschließenden Zeitintervall 16 auftretenden zweiten Druckdifferenz 19).

Insbesondere bei zwischenzeitlichen Messungen, also während des Inspirationsvorgangs 11 und vor Erreichen des inspiratorischen Spitzendruckes 25 bzw. während des Exspirationsvorgangs 12 und vor Erreichen des endexspiratorischen Druckes 26, sollte berücksichtigt werden, dass bei FCV insbesondere in der zweiten Hälfte 27 des Inspirationsvorgangs 11 und in der zweiten Hälfte 27 des Exspirationsvorgangs 12 (sehr) stabile bzw. konstante Fluidstrombedingungen und daher dann besonders vorteilhafte Messbedingungen vorliegen.

### Bezugszeichenliste

- 1: Beatmungsvorrichtung
- 2: Gaszuführeinrichtung
- 3: Gasabführeinrichtung
- 4: erster Fluidstrom
- 5: Atemweg
- 6: zweiter Fluidstrom
- 7: Umgebung
- 8: Drucksensor
- 9: Druck
- 10: Steuereinrichtung
- 11: Inspirationsvorgang
- 12: Exspirationsvorgang
- 13: erster Zeitpunkt
- 14: erste Druckdifferenz
- 15: erster Druck
- 16: Zeitintervall
- 17: zweiter Druck
- 18: zweiter Zeitpunkt
- 19: zweite Druckdifferenz
- 20: dritter Druck
- 21: vierter Druck
- 22: endexspiratorischer Zustand
- 23: endinspiratorischer Zustand
- 24: Verlauf
- 25: inspiratorischer Spitzendruck
- 26: endexspiratorischer Druck
- 27: zweite Hälfte
- 28: Zeit
- 29: Volumen
- 30: Visualisierungseinrichtung
- 31: Knickpunkt

## Patentansprüche

1. Beatmungsvorrichtung (1), zumindest umfassend eine Gaszuführeinrichtung (2) und eine Gasabführeinrichtung (3), zum Zuführen eines ersten Fluidstroms (4) hin zu einem Atemweg (5) eines Patienten und zum Abführen eines zweiten Fluidstroms (6) aus dem Atemweg (5) zurück in die Beatmungsvorrichtung (1) oder an eine Umgebung (7), einen Drucksensor (8) zur Erfassung eines Druckes (9) in dem Atemweg (5) sowie eine Steuereinrichtung (10) zum Betreiben der Beatmungsvorrichtung (1); wobei der Fluidstrom (4, 6) zumindest während eines Inspirationsvorgangs (11) und eines Exspirationsvorgangs (12) auf einen konstanten Wert einstellbar ist; **dadurch gekennzeichnet, dass** die Steuereinrichtung (10) zur Durchführung eines Verfahrens eingerichtet ist, das zumindest die folgenden Schritte umfasst:
a) Durchführen eines Inspirationsvorgangs (11) mit einem konstanten ersten Fluidstrom (4) mittels der Gaszuführeinrichtung (2),
b) Stoppen des ersten Fluidstroms (4) mittels der Gaszuführeinrichtung (2) zu einem ersten Zeitpunkt (13), und dabei
c) Bestimmen einer ersten Druckdifferenz (14) zwischen einem zum ersten Zeitpunkt (13) des Stoppens vorliegenden ersten Druck (15) und einem sich nach einem Zeitintervall (16) einstellenden zweiten Druck (17) mittels des Drucksensors (8); sowie
d) Durchführen eines Exspirationsvorgangs (12) mit einem konstanten zweiten Fluidstrom (6) mittels der Gasabführeinrichtung (3),
e) Stoppen des zweiten Fluidstroms (6) mittels der Gasabführeinrichtung (3) zu einem zweiten Zeitpunkt (18), und dabei
f) Bestimmen einer zweiten Druckdifferenz (19) zwischen einem zum zweiten Zeitpunkt (18) des Stoppens vorliegenden dritten Druck (20) und einem sich nach einem Zeitintervall (16) einstellenden vierten Druck (21) mittels des Drucksensors (8);
g) Festlegen und Bereitstellen einer Differenz aus der ersten Druckdifferenz (14) und der zweiten Druckdifferenz (19) als ein erster Kennwert, der zur Bestimmung zumindest einer gewebsrelatierten Resistance des Patienten verwendbar ist.

2. Beatmungsvorrichtung (1) nach Patentanspruch 1, wobei mit der Durchführung der Schritte d) bis f), und wenn der dritte Druck (20) einem endexspiratorischen Druck (26) entspricht, ein zweiter Kennwert festgelegt und bereitgestellt und damit eine atemwegsrelatierte Resistance des Patienten bestimmbar ist.

3. Beatmungsvorrichtung (1) nach einem der vorhergehenden Patentansprüche, wobei für Schritt g) festgelegt ist, dass die gewebsrelatierte Resistance im endexspiratorischen Zustand (22) vernachlässigbar und im endinspiratorischen Zustand (23) maximal ist und dazwischen während des Inspirationsvorgangs (11) linear zunimmt und während des Exspirationsvorgangs (12) linear abnimmt.

4. Beatmungsvorrichtung (1) nach Patentanspruch 3, wobei mit der Steuereinrichtung (10) zumindest zur Bestimmung der gewebsrelatierten Resistance eine Regressionsanalyse durchführbar ist.

5. Beatmungsvorrichtung (1) nach einem der vorhergehenden Patentansprüche, wobei der Drucksensor (8) endotracheal angeordnet ist.

6. Beatmungsvorrichtung (1) nach einem der vorhergehenden Patentansprüche, wobei mit der Steuereinrichtung (10) in Schritt g) auch ein zweiter Kennwert festgelegt und bereitgestellt ist und damit eine atemwegsrelatierte Resistance und durch Umrechnung auf den konstanten Fluidstrom (4, 6) der Druckfall in dem Atemweg (5) während des Inspirationsvorgangs (11) und des Exspirationsvorgangs (12) sowie ein alveolärer Druck (9) bzw. Verlauf (24) eines alveolären Druckes (9) bestimmbar ist.

7. Beatmungsvorrichtung (1) nach einem der vorhergehenden Patentansprüche, wobei zumindest der zweite Druck (17) oder der vierte Druck (21) rechnerisch bestimmbar ist.

8. Beatmungsvorrichtung (1) nach einem der vorhergehenden Patentansprüche, wobei zumindest der erste Zeitpunkt (13) in einer zeitlichen zweiten Hälfte (27) des Inspirationsvorgangs (11) oder der zweite Zeitpunkt (18) in einer zeitlichen zweiten Hälfte (27) des Exspirationsvorgangs (12) festgelegt ist.

9. Beatmungsvorrichtung (1) nach einem der vorhergehenden Patentansprüche, wobei zumindest in Schritt b) das Stoppen des ersten Fluidstroms (4) bei Erreichen eines festgelegten inspiratorischen Spitzendruckes (25) oder in Schritt e) das Stoppen des zweiten Fluidstroms (6) bei Erreichen eines festgelegten endexspiratorischen Druckes (26) erfolgt.

10. Beatmungsvorrichtung (1) nach Patentanspruch 9, wobei sich bei der ersten Druckdifferenz (14) eine (Gesamt-)Resistance aus einer Summe einer atemwegsrelatierten Resistance und einem Maximum einer gewebsrelatierten Resistance ergibt.

11. Beatmungsvorrichtung (1) nach Patentanspruch 9, wobei sich bei der zweiten Druckdifferenz (19) die (Gesamt-)Resistance aus der atemwegsrelatierten Resistance ergibt.

## Claims

1. Ventilator (1), at least comprising a gas supply device (2) and a gas discharge device (3), for supplying a first fluid flow (4) to an airway (5) of a patient and for discharging a second fluid flow (6) from the airway (5) back into the ventilator (1) or to an environment (7), a pressure sensor (8) for measuring a pressure (9) in the airway (5), and a control device (10) for operating the ventilator (1); wherein the fluid flow (4, 6) is adjustable to a constant value at least during an inspiration process (11) and an expiration process (12); **characterized in that** the control device (10) is configured to carry out a method comprising at least the following steps:
a) carrying out an inspiration process (11) with a constant first fluid flow (4) by means of the gas supply device (2),
b) stopping the first fluid flow (4) by means of the gas supply device (2) at a first time point (13), and at the same time
c) determining a first pressure difference (14) between a first pressure (15) present at the first time point (13) of stopping and a second pressure (17) occurring after a time interval (16) by means of the pressure sensor (8); and
d) carrying out an expiration process (12) with a constant second fluid flow (6) by means of the gas discharge device (3),
e) stopping the second fluid flow (6) by means of the gas discharge device (3) at a second time point (18), and at the same time
f) determining a second pressure difference (19) between a third pressure (20) present at the second time point (18) of stopping and a fourth pressure (21) occurring after a time interval (16) by means of the pressure sensor (8);
g) defining and providing a difference between the first pressure difference (14) and the second pressure difference (19) as a first index which is usable for determination of at least a tissue-related resistance of the patient.

2. Ventilator (1) as claimed in claim 1, wherein, by carrying out steps d) to f), and when the third pressure (20) corresponds to an end-expiratory pressure (26), a second index is defined and provided and an airway-related resistance of the patient is thus determinable.

3. Ventilator (1) as claimed in either of the preceding claims, wherein it is defined for step g) that the tissue-related resistance is negligible in the end-expiratory state (22) and maximal in the end-inspiratory state (23) and, in between, increases linearly during the inspiration process (11) and decreases linearly during the expiration process (12).

4. Ventilator (1) as claimed in claim 3, wherein a regression analysis is performable by means of the control device (10) at least to determine the tissue-related resistance.

5. Ventilator (1) as claimed in any of the preceding claims, wherein the pressure sensor (8) is arranged endotracheally.

6. Ventilator (1) as claimed in any of the preceding claims, wherein a second index is also defined and provided by means of the control device (10) in step g), and what are thus determinable are an airway-related resistance and, by conversion to the constant fluid flow (4, 6), the pressure drop in the airway (5) during the inspiration process (11) and the expiration process (12) and also an alveolar pressure (9) or plot (24) of an alveolar pressure (9).

7. Ventilator (1) as claimed in any of the preceding claims, wherein at least the second pressure (17) or the fourth pressure (21) is mathematically determinable.

8. Ventilator (1) as claimed in any of the preceding claims, wherein at least the first time point (13) is defined in a temporal second half (27) of the inspiration process (11) or the second time point (18) is defined in a temporal second half (27) of the expiration process (12).

9. Ventilator (1) as claimed in any of the preceding claims, wherein, at least in step b), the first fluid flow (4) is stopped when a defined peak inspiratory pressure (25) has been reached or, in step e), the second fluid flow (6) is stopped when a defined end-expiratory pressure (26) has been reached.

10. Ventilator (1) as claimed in claim 9, wherein, in the case of the first pressure difference (14), a (total) resistance arises from a sum total of an airway-related resistance and a maximum of a tissue-related resistance.

11. Ventilator (1) as claimed in claim 9, wherein, in the case of the second pressure difference (19), the (total) resistance arises from the airway-related resistance.

## Revendications

1. Dispositif respiratoire (1), comprenant au moins un dispositif d'alimentation en gaz (2) et un dispositif d'évacuation de gaz (3), pour alimenter un premier flux de fluide (4) vers une voie respiratoire (5) d'un patient et pour évacuer un second flux de fluide (6) de la voie respiratoire (5) vers le dispositif respiratoire (1) ou vers un environnement (7), un capteur de pression (8) pour détecter une pression (9) dans la voie respiratoire (5), ainsi qu'un dispositif de commande (10) pour faire fonctionner le dispositif respiratoire (1) ; dans lequel le flux de fluide (4, 6) est réglable à une valeur constante au moins pendant un processus d'inspiration (11) et un processus d'expiration (12) ; **caractérisé en ce que** le dispositif de commande (10) est agencé pour mettre en œuvre un procédé comprenant au moins les étapes suivantes :
a) effectuer un processus d'inspiration (11) avec un premier flux de fluide constant (4) à l'aide du dispositif d'alimentation en gaz (2),
b) arrêter le premier flux de fluide (4) à l'aide du dispositif d'alimentation en gaz (2) à un premier instant (13), tout en
c) déterminer une première différence de pression (14) entre une première pression (15) présente au premier instant (13) de l'arrêt et une deuxième pression (17) apparaissant après un intervalle de temps (16) au moyen du capteur de pression (B) ; et
d) effectuer un processus d'expiration (12) avec un deuxième flux de fluide constant (6) à l'aide du dispositif d'évacuation de gaz (3),
e) arrêter le deuxième flux de fluide (6) au moyen du dispositif d'évacuation de gaz (3) à un deuxième instant (18), et
f) déterminer une deuxième différence de pression (19) entre une troisième pression (20) présente au deuxième instant (18) de l'arrêt et une quatrième pression (21) apparaissant après un intervalle de temps (16) au moyen du capteur de pression (8) ;
g) définir et fournir une différence entre la première différence de pression (14) et la deuxième différence de pression (19) en tant que première valeur caractéristique pouvant être utilisée pour déterminer au moins une résistance tissulaire du patient.

2. Dispositif respiratoire (1) selon la revendication 1, dans lequel, avec l'exécution des étapes d) à f), et si la troisième pression (20) correspond à une pression expiratoire finale (26), une deuxième valeur caractéristique est définie et fournie et ainsi une résistance relative aux voies respiratoires du patient peut être déterminée.

3. Dispositif respiratoire (1) selon l'une quelconque des revendications précédentes, dans lequel, pour l'étape g), il est spécifié que la résistance relative au tissu est négligeable dans l'état expiratoire final (22) et maximale dans l'état inspiratoire final (23) et augmente de manière linéaire pendant le processus d'inspiration (11) et diminue de manière linéaire pendant le processus d'expiration (12).

4. Dispositif respiratoire (1) selon la revendication 3, dans lequel une analyse de régression peut être effectuée avec le dispositif de commande (10) au moins pour déterminer la résistance relative aux tissus.

5. Dispositif respiratoire (1) selon l'une quelconque des revendications précédentes, dans lequel le capteur de pression (8) est disposé de manière endotrachéale.

6. Dispositif respiratoire (1) selon l'une quelconque des revendications précédentes, dans lequel une deuxième valeur caractéristique est également définie et fournie avec le dispositif de commande (10) à l'étape g), ce qui permet de déterminer une résistance relative aux voies respiratoires et, par conversion au flux de fluide constant (4, 6), la chute de pression dans les voies respiratoires (5) pendant le processus d'inspiration (11) et le processus d'expiration (12), ainsi qu'une pression alvéolaire (9) ou l'évolution (24) d'une pression alvéolaire (9).

7. Dispositif respiratoire (1) selon l'une quelconque des revendications précédentes, dans lequel au moins la deuxième pression (17) ou la quatrième pression (21) peut être déterminée par calcul.

8. Dispositif respiratoire (1) selon l'une quelconque des revendications précédentes, dans lequel au moins le premier instant (13) est défini dans une seconde moitié temporelle (27) du processus d'inspiration (11) ou le second instant (18) est défini dans une seconde moitié temporelle (27) du processus d'expiration (12).

9. Dispositif respiratoire (1) selon l'une quelconque des revendications précédentes, dans lequel au moins à l'étape b), l'arrêt du premier flux de fluide (4) se produit lorsqu'une pression de pointe inspiratoire définie (25) est atteinte ou à l'étape e), l'arrêt du deuxième flux de fluide (6) se produit lorsqu'une pression expiratoire finale définie (26) est atteinte.

10. Dispositif respiratoire (1) selon la revendication 9, dans lequel, pour la première différence de pression (14), une résistance (totale) résulte d'une somme d'une résistance relative aux voies respiratoires et d'un maximum d'une résistance relative aux tissus.

11. Dispositif respiratoire (1) selon la revendication 9, dans lequel, pour la deuxième différence de pression (19), la résistance (totale) résulte de la résistance relative aux voies respiratoires.
